# EUROPEAN PATENT APPLICATION

(11) **EP 4 809 829 A1**
(43) Date of publication of application: **23.09.2026**
(21) Application number: 25197006.7
(22) Date of filing: 20.08.2025
(51) Int. Cl.: A23K 20/105, A23K 50/90, A23K 20/163

(54) **PREPARATION FOR FEEDING ANIMALS OF THE FAMILY APIDAE, BOMBYCIDAE OR SATURNIIDAE AND A COMPOSITION FOR USE IN THE TREATMENT OR PREVENTION OF NOSEMA AND OTHER DISEASES**

(30) Priority: 18.03.2025 PL 45150425
(71) Applicant: Bioscientia Sp. z o.o., 61-820 Poznan (PL)
(72) Inventor: Belter, Agnieszka, 62-023 Kamionki (PL); Skupi ska, Miros awa, 60-461 Pozna (PL)
(74) Representative: Kowalkiewicz, Zuzanna

(57) **Abstract**

An object of the invention is a preparation comprising a complex of metformin and cyclodextrin or/and a complex of metformin and heptakis(2,3,6-tris-O-methyl)cyclodextrin or/and metformin hydrochloride. Also an object of the invention is a veterinary composition, a feed additive for *Apidae, Bombycidae* or *Saturniidae,* and a non-therapeutic means for supporting abundance, post-wintering condition, number of broods, increasing productivity, increasing diversity and abundance of gut microbiome bacteria, activity of the immune system, supporting condition after contact with plant protection products, reducing morbidity of infections of *Apidae, Bombycidae* or *Saturniidae.*

## Description

The object of the invention is a preparation for feeding animals of the family *Apidae, Bombycidae* or *Saturniidae,* a composition for use in the treatment or prevention of nosema, and a feed additive. The invention also relates to a non-therapeutic way of supporting the number, condition after overwintering, number of larvae, increase productivity, increase diversity and abundance of gut microbiome bacteria, activity of the immune system, support of condition after contact with pesticides, reduction of the incidence of infections in animals of the family *Apidae, Bombycidae* or *Saturniidae.* The invention belongs to the field of veterinary medicine, including animal production.

In recent years, the phenomenon of mass extinction of *Apidae* has taken on an intensity and is a global problem. It primarily affects bees in highly industrialized countries such as the US, Canada, Brazil, Taiwan, England, Germany, Spain, Greece, and France. The weakening of bee colonies is a result of the invasion of parasites of these insects, the appearance of neonicotinoids in the environment causing a reduction in the immunity of bees, the overexploitation of insects in industrialized apiaries, changes in the environment (monocultures) causing a reduction in the number of plants that provide food for bees, which is caused, among other things, by the global intensification of agriculture. This is also caused by the observed decline in the population of free-living bumblebees. The multifaceted nature of the problem of the health of these insects makes it increasingly necessary to use measures to support the health and thus productivity of bees and bumblebees.

Honey bees *(Apis mellifera)* are food producers, so drugs and feeds dedicated to them must meet the highest safety standards. Currently, the number of drugs and pharmacologically active preparations on the market is small and does not cover consumer demand. The number of active substances in medicines for bees that meet the requirements of EU Directives 2001/110/EC and 2014/63/EU, Regulation No. 470/2009 and No. 37/2010 counts only a few items: amitraz, coumaphos, flumethrin, oxalic acid, and taufluvalinate. The legislation mandates the use of preparations that are safe for humans and the environment. In addition, it restricts the use of antibiotics in veterinary medicine, which was the reason for the ban on fumagillin, an effective drug in the control of *Nosema ceranae,* in EU countries. Often, the only remedies for the nosema-causing parasite in an apiary are the destruction of the bee colony, disinfection of hives and tools, feeding, and the supportive use of herbal mixtures with incompletely understood effects. Market demand for new preparations related to improving the health of bees is therefore enormous, as also indicated by numerous reports and studies by the European Commission and the European Medicines Agency, as well as by the opinions of beekeepers themselves. District and provincial veterinarians - the bodies responsible for monitoring and controlling bee diseases.

Of the approximately 250 to 300 species of bumblebees *(Bombycidae)* in the world, several are now being bred commercially on a mass scale and distributed to pollinate plants. Screening studies have shown that many of these commercially reared bumblebees contain parasites such as *Nosema* spores. Bumblebee colonies infected with this sporocarps produce colonies of smaller size and fewer sexual offspring. There are real concerns that higher infection rates among commercial colonies could lead to the transmission of *N. bombi* to wild bumblebee populations. In addition, *Nosema* sporozoan infections in bumblebee populations have been linked to climate change, with a higher incidence of infection occurring in warmer and drier years.

Bees and bumblebees are not the only ones at risk of nosema. Silkworm microsporidiosis of *Bombyx mori L.* is one of the most dangerous diseases caused by a parasite belonging to the genus *Nosema.* Infections with this disease can be chronic or highly virulent and can result in total losses to the silk farming industry. *Nosema bombycis* was first described in 1857 and caused the annihilation of the French and Italian silk industry in 1865. To this day, it is still considered a major risk in silkworm production systems and is becoming increasingly complex as more strains/species of microsporidia infecting silkworms are identified worldwide (Bhat, Shabir & Bashir, Ifat & Kamili, Afifa. (2009). Microsporidiosis of silkworm, Bombyx mori L. (Lepidoptera bombycidae): A review. Afr J Agric Res. 4.). Another organism exposed to the *Nosema* sporozoan is the silkworm, *Antheraea pernyi.* The silkworm is native to the area of present-day China, but due to domestication it can be found almost all over the globe. *Antheraea pernyi* is used in the production of silk (genus *tussar).* For these reasons, the serious impact of *Nosema* parasites in silk production requires hygienic measures and the housing of eggs and early stages of silkworms in specialized, well-equipped facilities to reduce the risk of infection.

The purpose of the invention was to develop a preparation for the feeding of animals of the family *Apidae, Bombycidae* or *Saturniidae,* a composition for use in the treatment or prevention of *nosema* caused by a sporozoan of the genus *Nosema* in these animals, and a feed additive for these animals. The invention also aimed to develop a non-therapeutic method that supports abundance, post-wintering condition, number of larvae, increase productivity (in the case of animals of the *Apidae* family, it is called honey yield), support condition after contact with plant protection products, increase the diversity and abundance of bacteria of the intestinal microbiome, activity of the immune system, reduce the incidence of infections in animals of the *Apidae, Bombycidae* or *Saturniidae* family.

Unexpectedly, it turned out that a veterinary preparation for feeding animals of the family *Apidae, Bombycidae* or *Saturniidae* containing metformin or its physiologically acceptable complex or salt can be used in the diet of these animals. Also unexpectedly, a veterinary composition whose active ingredient is metformin or its veterinary acceptable complex or salt can be used in the treatment or prevention of nosema of animals of the family *Apidae, Bombycidae* or *Saturniidae* caused by the sporocarp of the genus *Vairimorpha (Nosema) apis. Vairimorpha (Nosema) ceranae, Nosema bombycis, Nosema bombi* or *Nosema pernyi.* Also unexpectedly, it turned out that a feed additive according to the invention for animals of the family *Apidae, Bombycidae* or *Saturniidae* containing metformin or its physiologically acceptable complex or salt could find use in the diet of these animals. The preparation, veterinary composition and additive are furthermore safe for insects, the environment and humans.

Unexpectedly, it was also found that a preparation containing metformin or its physiologically acceptable complex or salt has a positive effect on abundance, post-wintering condition, larvae number, productivity (in the case of *Apidae,* this is referred to as honey yield), support of condition after contact with pesticides, increases the diversity and abundance of bacteria of the intestinal microbiome, activity of the immune system, and reduces the incidence of infections in *Apidae, Bombycidae* or *Saturniidae.*

Metformin is an effective and safe active pharmaceutical substance. Its use has many metabolic benefits in humans, including lowering blood glucose levels and inhibiting gluconeogenesis. This is consistent with the description of metformin's multidirectional effects on the metabolism of cells of various organs in other animals studied. One of the most important actions of metformin is the activation of an important enzyme (AMPK) that increases the uptake of glucose by the body's cells and leads to lipid oxidation to maintain proper energy balance in the body. Glucose homeostasis throughout the bee's body has a positive effect. In addition, metformin has action to stimulate the microbiome, which can also be a phenomenon that affects the condition of bees. The substance also exhibits anti-inflammatory effects.

The well-being of *Apidae, Bombycidae* and *Saturniidae* and their productivity largely depend on the condition of their intestinal epithelium. *Nosema* sporozoite infections are often accompanied by intestinal cell damage, which in turn causes a decline in the condition of these insects and their increased mortality. Metformin or its physiologically acceptable complex or salt increases the viability of progenitor cells isolated from the eggs of these insects, particularly the honeybee. Metformin or its physiologically acceptable complex or salt, by stimulating the development of progenitor cells, can affect the recovery of the intestinal epithelium, thereby influencing the condition of these insects, supporting their resistance to infection and other harmful agents. In addition, metformin or its physiologically acceptable complex or salt has been shown to reduce the level of oxygen free radicals in the hemolymph and increase the level of superoxide dismutase, which is crucial in alleviating the oxidative stress accompanying infections. The undoubted advantage of metformin or its physiologically acceptable complex or salt is that it is safe for human use (it has been used successfully in clinical settings).

If a particular protection method works favorably on the honey bee, it is highly likely to work on other wild pollinators as well. This is due to the physiological similarity of insects belonging to the same order. The order *Hymenoptera (Hymenoptera)* includes both well-known and ecologically important insects, such as bees, wasps and ants, and lesser-known ones, such as the pilot bees, caterpillars, bleschers and bumblebees. Their evolutionary history dates back to the Carboniferous period, with most of today's major groups appearing in the Jurassic. The order is divided into two distinct suborders: the herbivores (*Symphyta*) and the styliforms *(Apocrita).*

Wild pollinators include various species of solitary bees, such as mason bees *(Osmia* spp.), bee bees *(Megachilidae),* proper bees *(Andrenidae),* rattlesnakes *(Xylocopa* spp.) and numerous species of bumblebees *(Bombus* spp.). In addition to bees, other hymenoptera, such as dome wasps ( *Vespidae),* which, although they feed on insects, adults often visit flowers in search of nectar, are also important pollinators.

Substances that exhibit beneficial effects on the physiological mechanisms of the honeybee often exert similar positive effects on other species of *hymenoptera.* This regularity is due to the close evolutionary affinity and common biochemical pathways characteristic of the order *Hymenoptera.* Over millions of years of evolution, since the emergence of the first hymenopterans in the Carboniferous, they developed similar immune mechanisms, metabolism regulation systems and detoxification pathways of xenobiotics.

It is particularly interesting to note that despite the enormous diversity of life forms and adaptive strategies within the *Hymenoptera* - from solitary wild bees to social ants to parasitic caterpillars - the basic physiological processes remain remarkably similar in them (Peters et al., 2017). This evolutionary inheritance means that preparations to boost immunity or improve the condition of the honeybee can be equally effective in other representatives of *hymenoptera.*

The essence of the invention is a preparation for feeding animals of the family *Apidae, Bombycidae* or *Saturniidae* containing metformin or its physiologically acceptable complex or/and salt.

In a preferred embodiment of the invention, it contains a complex of metformin and cyclodextrin or/and a complex of metformin and heptakis(2,3,6-tris-O-methyl)cyclodextrin or/and metformin hydrochloride.

In a preferred embodiment of the invention, animals belong to the genus *Apis, Bombus, Psithyrus, Bombyx, Antheraea,* favorably animals of the species *Apis melifera, Apis cerana, Apis dorsata, Apis florea, Bombyx mori L., Antheraea pernyi.*

In a preferred embodiment of the invention, it contains at least one attractant selected from the group consisting of: bee pollen, royal jelly, honey, feather, sucrose solution, fructose solution, glucose solution, sugar syrup, invert syrup, sugar paste, honey-sugar cake (cand.).

In a preferred embodiment of the invention, the concentration of metformin in its physiologically acceptable complex or/and salt is up to 6.5 g metformin/dm³ of liquid attractant or up to 6.5 g metformin/kg of solid attractant.

The essence of the invention is a veterinary composition characterized in that the active ingredient is metformin or a veterinary acceptable complex or/and salt thereof for use in the treatment or prevention of nosema in animals of the family *Apidae, Bombycidae* or *Saturniidae* caused by the sporocarp *Vairimorpha (Nosema) apis. Vairimorpha (Nosema) ceranae, Nosema bombycis, Nosema bombi* or *Nosema pernyi.*

In a preferred embodiment of the invention, the active substance is a complex of metformin and cyclodextrin or/and a complex of metformin and heptakis(2,3,6-tris-O-methyl)cyclodextrin or metformin hydrochloride.

In a preferred embodiment of the invention, animals belonging to the genus *Apis, Bombus, Psithyrus, Bombyx, Antheraea,* favorably animals of the species *Apis melifera, Apis cerana, Apis dorsata, Apis florea, Bombyx mori L., Antheraea pernyi.*

In a preferred embodiment of the invention, the concentration of the active substance is up to 6.5 g metformin/dm³ of liquid attractant or up to 6.5 g metformin/kg of solid attractant.

In a preferred embodiment of the invention, the acceptable carrier is at least one ingredient selected from the group consisting of: bee pollen, royal jelly, honey, feather, sucrose solution, fructose solution, glucose solution, sugar syrup, invert syrup, sugar paste, honey-sugar cake (cand.).

The essence of the invention is a feed additive for animals of the family *Apidae, Bombycidae* or *Saturniidae* containing metformin or/and its physiologically acceptable complex or salt.

In a preferred embodiment of the invention, it contains a complex of metformin and cyclodextrin or/and a complex of metformin and heptakis(2,3,6-tris-O-methyl)cyclodextrin or metformin hydrochloride.

In a preferred embodiment of the invention, animals belonging to the genus *Apis, Bombus, Psithyrus, Bombyx, Antheraea,* favorably animals of the species *Apis melifera, Apis cerana, Apis dorsata, Apis florea, Bombyx mori L., Antheraea pernyi.*

In a preferred embodiment of the invention, it contains at least one attractant selected from the group consisting of: bee pollen, royal jelly, honey, feather, sucrose solution, fructose solution, glucose solution, sugar syrup, invert syrup, sugar paste, honey-sugar cake (cand.).

In a preferred embodiment of the invention, the concentration of metformin in its physiologically acceptable complex or salt is up to 6.5 g metformin/dm³ of liquid attractant or up to 6.5 g metformin/kg of solid attractant.

The essence of the invention is a non-therapeutic way to support counts, post-wintering condition, larvae counts, increase productivity, increase diversity and abundance of gut microbiome bacteria, immune system activity, support condition after contact with pesticides, reduce the incidence of infections in animals of the family *Apidae, Bombycidae* or *Saturniidae* by feeding them with a preparation according to the invention, a veterinary composition according to the invention or a feed additive according to the invention.

In a preferred embodiment of the invention, a preparation, composition or additive is administered once or repeatedly.

In a preferred embodiment of the invention, it increases the honey yield of *Apidae.*

The salt of the metformin compound in this application may mean a physiologically or veterinary acceptable salt from among salts that are substances in which the cation and anion are linked by electrostatic attraction. For example, the salt may be at least one selected from the group including metal salts, salts with organic bases, salts with inorganic acids, salts with organic acids, salts with basic or acidic amino acids and the like. In one embodiment, the metal salt may be at least one selected from the group including alkali metal salts (sodium salt, potassium salt, etc.), alkaline earth metal salts (calcium salt, magnesium salt, barium salt, etc.), aluminum salts, and the like.), aluminum salts and the like; and the salt with organic bases may be at least one selected from the group including salts with triethylamine, pyridine, picoline, 2,6- rutidine, ethanolamine, diethanolamine, triethanolamine, cyclohexylamine, dicyclohexylamine, *N,N*-dibenzylethylenediamine and the like; and the salts with inorganic acids may be at least one salt selected from the group consisting of hydrochloric acid, hydrobromic acid, nitric acid, sulfuric acid, phosphoric acid and the like; and the salt with organic acids may be at least one salt selected from the group including salts with formic acid, acetic acid, trifluoroacetic acid, phthalic acid, fumaric acid, oxalic acid, tartaric acid, maleic acid, citric acid, succinic acid, methanesulfonic acid, benzenesulfonic acid, p-toluenesulfonic acid and the like; and the salt with basic amino acids may be at least one salt selected from the group consisting of salts with arginine, lysine, ornithine and the like; and the salt with acidic amino acids may be at least one salt selected from the group consisting of salts with aspartic acid, glutamic acid and the like.

The salt of the metformin compound in this application may mean a physiologically or veterinary acceptable salt from among the salts that are substances in which the cation and anion are linked by electrostatic attraction. For example, metformin may be administered as one of the pharmaceutically or physiologically acceptable salts, such as hydrochloride, acetate, benzoate, citrate, fumarate, embonate, chloropheno-xyacetate, glycolate, palmitate, aspartate, methanesulfonate, maleate, para-chlorophenoxy-isobutyrate, formate, lactate, succinate, sulfate, tartrate, cyclohexanecarboxylate, hexanoate, octanoate, decanoate, hexadecanoate, octadecanoate, benzenesulfonate, trimethoxybenzoate, paratoluenesulfonate, adamantanecarboxylate, malate, glutamate, pyrrolidonecarboxylate, naphthalenesulfonate, 1- glucosephosphate, nitrate, sulfite, dithionate or phosphate. Particularly preferred salts among the above are hydrochloride, fumarate, embonate and chlorophenoxyacetate. The metformin salts used in pharmaceuticals are obtained by the state-of-the-art known method, by acting metformin on a suitable acid.

The complex of the metformin compound in the present application may mean a physiologically or veterinary acceptable complex from among complexes that are substances in which at least one central atom, surrounded by other atoms or groups of atoms, called ligands, with at least one bond of the central atom to the ligand being a coordination bond. The metformin compound complex in this application may also mean a physiologically or veterinary acceptable complex among the complexes, which are inclusion compounds (also known as inclusion complexes) are intermolecular chemical combinations of two or more components, formed by the confinement of molecules (atoms, ions) of one component. For example, the host can be at least one selected from the group including cyclodextrin, heptakis(2,3,6-tris-O- methyl)cyclodextrin, α-cyclodextrin, β-cyclodextrin, γ-cyclodextrin. A special case of inclusion compounds are so-called clathrates, in which the host structure is closed (so-called cage). In such structures the role of the host is often played by fullerenes (an allotropic variety of carbon), then superconducting materials can be formed. Another group of inclusion compounds is also formed by zeolites, i.e. minerals with a porous structure that is a complex network of tunnels and cages, in which other substances - mainly gases and liquids - can accumulate. They are often used for this purpose. Inclusion structures are often formed by crystalline organic compounds.

### Figure description.

**Fig. 1** - Results of analyses of the effects of metformin and its complexes on metabolic (A) and proliferative (B) activity of cells of the L929 line. The level of statistical significance of the obtained results was tested using one-way analysis of variance (p < 0.05)
**Fig. 2** - Effect of metformin (MF) and its complex with heptakis(2,3,6-tris-O- methyl)cyclodextrin (MF-ME) on the proliferative activity of cells isolated from bee eggs. Statistical analysis of the data was performed using a one-way ANOVA test. Statistically significant differences are marked with an asterisk (*p < 0.05), statistically insignificant differences are marked with *ns*
**Fig. 3** - Effect of metformin (MF) and its complex with heptakis(2,3,6-tris-O- methyl)cyclodextrin (MF-ME) on the metabolic activity of cells isolated from bee eggs. Statistical analysis of the data was performed using a one-way ANOVA test. Statistically significant differences are marked with an asterisk (*p < 0.05; ***p < 0.001), statistically insignificant differences are marked with *ns*
**Fig. 4** - Effect of metformin on the metabolic activity of honeybee-derived cells
**Fig. 5** - Effect of different culture conditions on mRNA expression for vitellogenin (VTG) and the receptor for vitellogenin (VTGR) in bee eggs
**Fig. 6** - Results of mitochondrial membrane potential analysis of bee cells isolated from eggs. Statistical analysis of the data was performed using a one-way ANOVA test. Statistically significant differences are marked with an asterisk (**p< 0.01; ***p< 0.001), statistically insignificant differences are marked with *ns*
**Fig. 7** **-** Transcript levels of pluripotency genes. Statistical analysis of the data was performed using one-way ANOVA test. Statistically significant differences are marked with an asterisk (*p < 0.05; **p < 0.01; ***p < 0.01), statistically insignificant differences are marked with *ns.* Differences between MF and MF-ME samples are marked in red, analysis was performed using Student's t-test
**Fig. 8** - Transcript levels of genes involved in apoptosis. Statistical analysis of the data was performed using one-way ANOVA test. Statistically significant differences are marked with an asterisk (*p< 0.05; **p< 0.01; ***p< 0.01), statistically insignificant differences are marked with *ns.* Differences between MF and MF-ME samples are marked in color, analysis was performed using Student's t-test
**Fig. 9** - Images obtained using scanning electron microscopy (top panel, 40 µm scale) and transmission microscopy (4 µm scale)
**Fig. 10** - Images obtained for cultures stained with calcein (top panel) and propidium iodide (50 µm scale)
**Fig. 11** - Schematic of experiments conducted in under action of preparation on the bee survival rate, prevention and control of honey bee nosema
**Fig. 12** **-** Bee survival rate by food group
**Fig. 13** **-** Abecin expression levels by food group
**Fig. 14** **-** Expression levels of defensin in different food groups
**Fig. 15** **-** Hymenoptecin expression levels in different food groups
**Fig. 16** - Bee survival rates by food group
**Fig. 17** - Level of nosema infection observed in each food group
**Fig. 18** - Abecin expression levels in different food groups
**Fig. 19** - Expression levels of defensin in different food groups
**Fig. 20** - Hymenoptecin expression levels in different food groups
**Fig. 21** - Bee survival rates by food group
**Fig. 22** - Level of nosema infection observed in each food group
**Fig. 23** - Abecin expression levels in different food groups
**Fig. 24** - Expression levels of defensin in different food groups
**Fig. 25** - Hymenoptecin expression levels in different food groups
**Fig. 26** - Results of multiplex PCR reaction for the presence of *DWV virus.* The markers above the gel correspond to: "M" - GeneRuler DNA Ladder Mix size marker (Thermo Fisher Scientific), "-" - control PCR reaction without matrix addition, and the numbers of subsequent samples tested. The red box indicates the expected size of the *DWV-specific* PCR fragment
**Fig. 27** **-** Results of multiplex PCR reaction for *BQCV.* The markers above the gel correspond to: "M" - GeneRuler DNA Ladder Mix size marker (Thermo Fisher Scientific), "-" - control PCR reaction without matrix addition, and the numbers of subsequent samples tested. The red box indicates the expected size of the *BQCV-specific* PCR fragment
**Fig. 28** - Results of multiplex PCR reaction for parasitic protozoa. The markers above the gel correspond to: "M" - size marker of GeneRuler DNA Ladder Mix (Thermo Fisher Scientific), "-"control PCR reaction without matrix addition, and the numbers of subsequent samples tested. The red boxes indicate the expected size of PC fragments

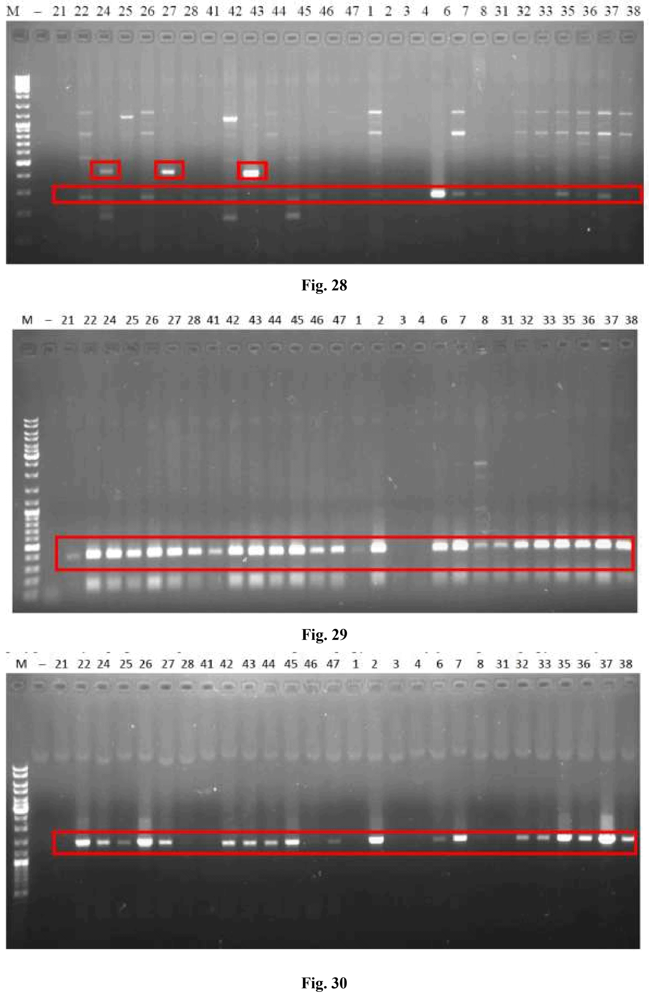

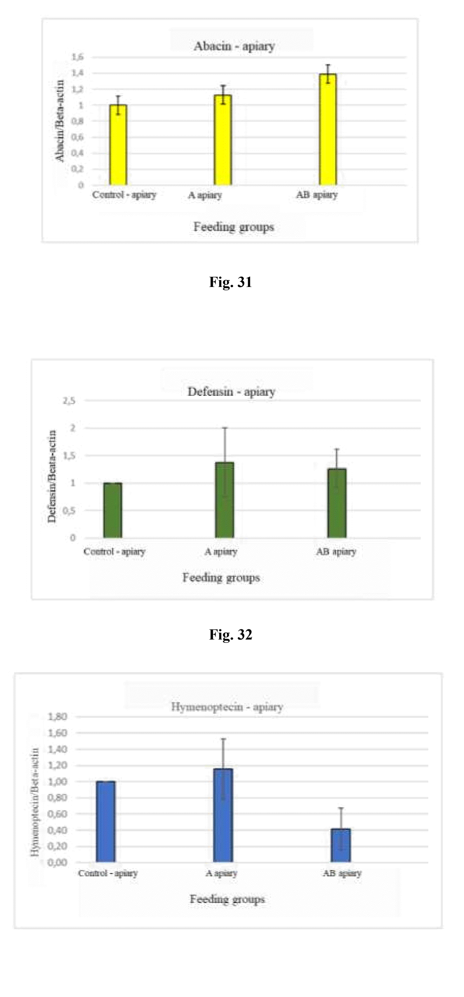

**Fig. 29** - Results of PCR reaction for the presence of *A. apis.* The markers above the gel correspond to: "M" - size marker of GeneRuler DNA Ladder Mix (Thermo Fisher Scientific), "-"control PCR reaction without matrix addition, and the numbers of subsequent samples tested. The red box indicates the expected size of the PCR fragment **Fig. 30** - Results of PCR reaction for the presence of *P. larvae.* The markers above the gel correspond to: "M" - size marker of GeneRuler DNA Ladder Mix (Thermo Fisher Scientific), "-" control PCR reaction without matrix addition, and the numbers of subsequent samples tested. The box indicates the expected size of the PCR fragment **Fig. 31** **-** Abacin expression levels in different food groups
**Fig. 32** **-** Expression levels of defensin in different food groups
**Fig. 33** **-** Hymenoptecin expression levels in different food groups
**Fig. 34** **-** Survival rate of bees in research groups in tact of cage experiment
**Fig. 35** - Nosema infection in bees from winter settlements
**Fig. 36** - Nosema infection in bees from winter settlements

The invention will be further illustrated with the following performance examples and on the figures of the drawing.

### Example I - Method of obtaining metformin and its complexes Met-CD and Met-ME

Methods of obtaining metformin (1,1-dimethylbiguanide) are known in the state of the art (e.g. Oxley and Short, 1995, Moghimi et al., 2011, Veisi et al., 2014, application WO2017044720). Met-CD and Met-ME complexes were obtained by methods known in the state of the art, e.g. Kaur et al, 2019 (Synthesis, characterization and studies on host-guest interactions of inclusion complexes of metformin hydrochloride with β-cyclodextrin, Journal of Molecular Liquids, vol. 282, pp. 162 - 168 (DOI: 10.1016/j.molliq.2019.02.127), Khaled et al, 2012 (Miniaturized ionophore-based potentiometric sensors for the flow-injection determination of metformin in pharmaceutical formulations and biological fluids, Analyst, 2012,137, 5680- 5687 (DOI: 10.1039/C2AN35696A)).

### Example II - Effects of metformin and its Met-CD and Met-ME complexes on the metabolic activity of cells, including mouse fibroblasts and bee cells - in vitro tests - on cells

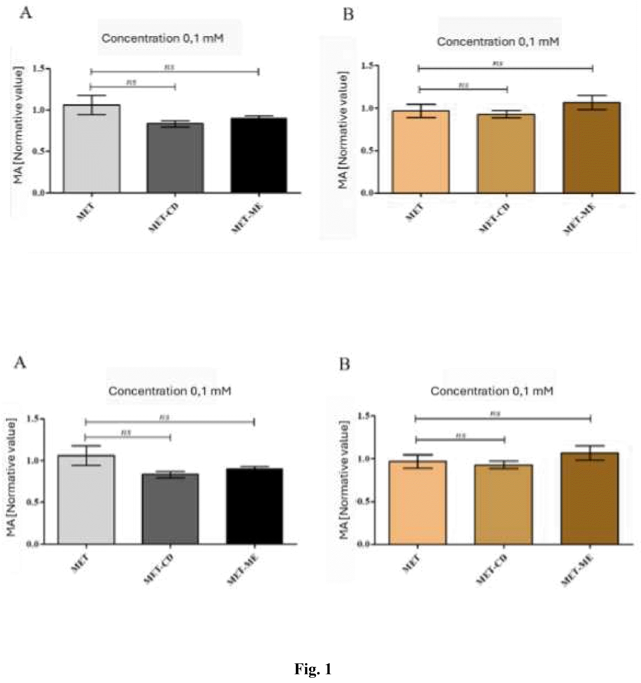

**II.A** The results of analyses of the effects of metformin and its complexes Met-CD and Met-ME against mouse fibroblast cells (line L929), where physical mixtures of Met- ME and Met-CD were used on metabolic and proliferative activity, confirmed the positive activity of these systems. *In vitro* tests showed that Met-ME and Met-CD are not cytotoxic to cells of the L929 line. In addition, studies indicate that Met and its complexes with β-cyclodextrin and with heptakis(2,3,6-tris-O-methyl)cyclodextrin at a concentration of 0.1 mM show similar biological effects - both in terms of their effects on metabolic and proliferative activity of fibroblasts **(****Fig. 1****).**
**I.B** An evaluation of the effect of the produced Met-ME and Met- CD complexes on the metabolic activity of progenitor cells isolated from honeybee eggs was also carried out, in particular, the evaluation was carried out with the assessment of the effect of the tested compounds on:
   1) proliferative activity (bromodeoxyuridine/BrdU incorporation assay into newly formed DNA strands), and
   2) Mitochondrial activity - Alamar Blue test and mitochondrial membrane potential analysis.

Metabolic activity studies were conducted using a model of progenitor cells isolated from honeybee eggs.

### The procedure for preparing eggs for testing included the following steps

### 1. Collecting bee eggs

The bee patches were delivered to the laboratory within 30 to 40 minutes of being pulled from the bee nest. The bee slices were obtained from a single bee colony and contained eggs laid by an isolated queen mother within the last 2 hours. Eggs were selected from the bee cells using long wooden sticks. 50-60 eggs were placed in 1.5 ml polypropylene conical tubes (eppendorf tubes; Capp). To avoid mechanical damage, the eggs were deposited on the walls of the tube.

### 2. Disinfection of bee eggs

After collection, bee eggs were disinfected using 70% high-purity ethanol (Merck/Sigma Aldrich, Molecular Biology Alcohol). The 70% alcohol was prepared using 99.8% alcohol and Hanks' salt free of magnesium and calcium ions (HBSS/Hank's Balanced Salt Solution, Sigma- Aldrich) with 2% antibiotic (PSA; Sigma Aldrich). Each batch was washed with 1 ml of ethanol; the incubation time for the eggs in the 70% ethanol solution was 5 minutes. After this time, eggs were collected with a pipette and transferred to a 15 ml centrifuge tube (Falcon type; Greiner), then centrifuged for 3 minutes at 300×g. Eggs that did not detach from the eppendrof type tube were washed again using 1 ml of 70% ethanol for 5 minutes. The activity was repeated three times. After a series of washes, the disinfected eggs were combined by suspending them in culture medium, but not exceeding a volume of 2 ml. The culture medium was *Dulbecco's Modified Eagle's Medium - high glucose*/*DMEM* HG, Sigma Aldrich) supplemented with 10% bovine serum (FBS; Sigma Aldrich L) and 2% antibiotic solution (PSA). The culture medium supplemented in this manner was referred to as *complete growth medium* (CGM).

### 3. Preparation of eggs for the experiment

Rinsed and disinfected eggs were suspended in culture medium (CGM). Eggs were thoroughly mixed by repipetting several times, then portioned into eppendorf-type tubes, preserving the initial number of eggs (i.e. 50-60 eggs) in the tube for the planned assay.

### 4. Preparation of compounds for testing

The results of bioassays showed that Met-Me complexes at concentrations equal to 0.1 mM exhibit the desired biological activity associated with an increase in cell proliferative activity. Given the data collected in screening assays carried out using a mouse fibroblast line (L929), metformin compounds and its Met-ME complexes at a concentration of 0.1 mM were tested in studies conducted using a bee cell model. The compounds were dissolved in CGM culture medium, at a final concentration of 8 mM. The control of the experiment was egg culture in CGM without the addition of the tested active substances.

### 5. Experimental cultures

Egg cultures were incubated with the test compounds for 2 hours at a constant temperature of 32°C, 90% humidity and 5% CO₂. After the experiment was completed, the eggs were centrifuged (3 min/300×g), then washed with Hanks salt containing no magnesium or calcium ions. The cultures were centrifuged again (3 min/300×g), after centrifugation the supernatant was removed. Eggs were resuspended in 550 µl of CGM and homogenized using a polypropylene hand-held micro-homogenizer (Sigma Aldrich). Homogenization was carried out until a homogeneous cell suspension was obtained. The cells were then transferred in a volume of 180 µl to three wells of a microtiter plate (96-well). Each assay was performed in triplicate.

### 5.1. Analysis of the effects of the tested compounds on proliferative activity

Proliferative activity was assessed using bromodeoxyuridine (BrdU), which is a synthetic thymidine analog that is incorporated into newly formed DNA, during its replication in the S phase of the cell cycle. Determination of the amount of BrdU in cells was carried out by ELISA technique using the BrdU Cell Proliferation ELISA Kit (Abcam, assay sensitivity 40 cells/well). The kit consists of rdU (500× concentrated), solution to fix the cells, anti-BrdU antibody, reaction inhibition buffer, goat anti-mouse IgG (anti-IgG)-conjugated peroxidase antibody (2,000 ×), buffer to allow dilution of the secondary antibody, substrate (tetramethylbenzidine, TMB), plate wash buffer (50×).

The procedure for determining cell proliferation activity using the BrdU Cell Proliferation ELISA Kit was performed according to the manufacturer's protocol. After seeding the cells, 20 µl of BrdU solution (1× concentrated prepared in CGM) was added to three wells, CGM (control "BrdU -") was added to another three wells. In the next three wells, 180 µl of CGM without cells was added and 20 µl of 1×-concentrated BrdU solution was added (control "BrdU +"). After the addition of BrdU, the cultures were incubated for 24 hours in a CO₂ incubator (32°C; 90% humidity; 5% CO₂). After incubation, the cultures (plates) were centrifuged for 5 minutes and at 300×g, then the culture supernatant was removed and 200 µl of fixative solution was added to each well. The incubation time of the cultures with the fixative was 30 minutes. The cultures were then dried, washed three times using 1× concentrated wash buffer. After washing, 100 µl of the I-strand antibody (anti-BrdU) was added to each well. The cultures were incubated for 1 hour at room temperature. After incubation, the cultures were washed three times with 1× concentrated wash buffer. The cultures were then incubated for 30 minutes at room temperature with peroxidase-conjugated antibody II. A solution containing 1× concentrated antibody was prepared according to the manufacturer's recommendation in the buffer provided with the kit. Before use, the antibody solution was filtered through a syringe filter (PVDF membrane with a porosity of 0.22 µm; Sigma Aldrich).

After incubation of the cultures with the second-strand antibody, the culture plates were washed three times with rinse solution and once with deionized water. Then 100 µl of substrate (TMB) was added to each well, and the cultures were incubated for 30 minutes at room temperature, in the dark - avoiding exposure of the plate to light. Then 100 µl of reaction inhibition solution was added to each well. Absorbance was measured at 450 nm and a reference wavelength of 550 nm . The analysis was performed using a reader Microplate Spectrophotometer (Epoch^{™} Microplate Spectrophotometer, BioTek). Proliferative activity was assessed by spectrophotometric measurement(ΔA=A450 - A550) of the normalized value calculated according to the formula: ΔAtcst sample = ΔAcontrolBrdU- - ΔAcontrolBrdU+.

### 5.2. Analysis of metabolic activity

### 5.2. 1. Alamar blue test

After seeding the cells, 20 µl of Alamar Blue dye (Sigma Aldrich) was added to each well. Alamar Blue contains an oxidation-reduction indicator and uses natural. The assay is based on the reduction of blue resazurin to red resorufin and takes advantage of metabolic processes in cells. Cells were incubated with dye for 2 hours (32°C; 90% humidity; 5% CO₂), then the supernatant was collected and transferred to a new microtiter plate for spectophotometric measurement at 600 nm and a reference wavelength of 660 nm. The CGM medium with added dye constituted the blank of the experiment). Analysis was performed using a microplate reader (Epoch^{™} Microplate Spectrophotometer, BioTek). Proliferative activity was assessed by spectrophotometric measurement, the normalized value calculated according to the formula:
ΔAblank- App of the test sample.

### 5.2.2. Assessment of mitochondrial membrane potential

Mitochondrial membrane activity analysis was performed using the Muse^{®} Mitopotential Assay Kit (Merck). The kit includes the following reagents: Dye: Muse^{®} MitoPotential Dye, Muse^{®} MitoPotential 7-AAD dye, 1× buffer (Assay Buffer).

The staining procedure was carried out according to the manufacturer's recommendations. After experimental culture with the test compounds, the cells were homogenized (according to the protocol described above). Before the determination of mitochondrial membrane activity, the cells were swirled (3 min/300×g), then washed with HBBS and swirled again (3 min/300×g). The cells were then resuspended in 100 µl of the buffer provided with the kit. Subsequently, the cells were stained using Muse^{®} MitoPotential Dye. The staining solution was prepared according to the manufacturer's recommendations at a ratio of 1:1,000 in lysis buffer. 95 µl of MitoPotential Dye was added to the cell suspension, then the cells were incubated for 20 minutes in a CO2 incubator (37°C; 90% humidity; 5% _{CO2}). After incubation, cells were stained by adding 5 µl of 7-AAD dye. Cells were incubated for 5 minutes at room temperature. Mitochondrial membrane activity was determined using a Muse cell analyzer^{®} Cell Analyzer.

### Results

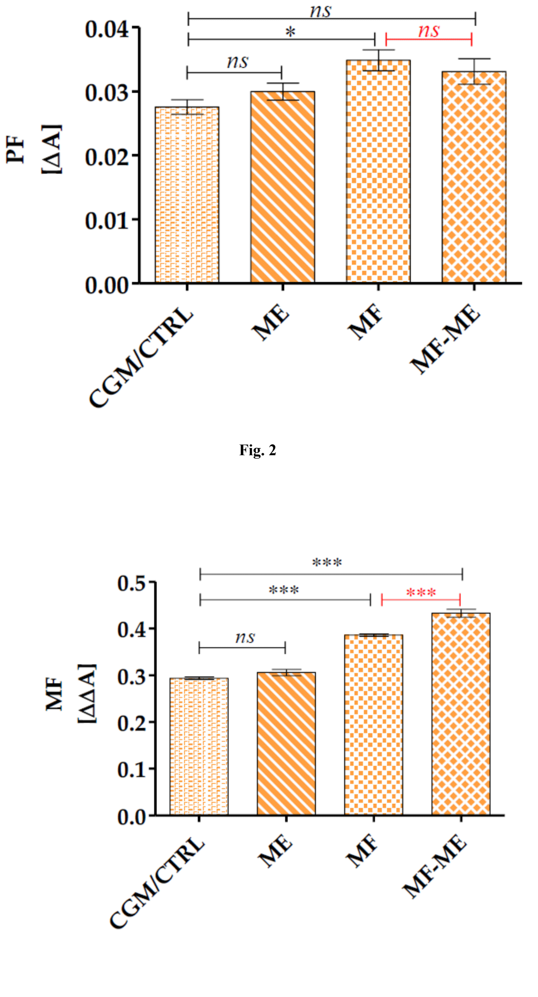

Analysis of the proliferative activity of the experimental cultures relative to the control culture (CGM) showed that each of the compounds tested enhances cell proliferative activity. However, a statistically significant difference was noted only in cultures treated with 0.1 mM metformin. In addition, the analysis showed that the biological activity of pure Met as well as its Met-ME complex in the context of modulating proliferative activity is similar **(****Fig. 2****).**

The results of the Alamar Blue test indicate that both metformin and its complex with ME significantly undermine metabolic activity in cultures of cells isolated from bee eggs. ME showed no cytotoxic effect against cells isolated from bee eggs. The study also showed that complexing metformin with ME increased its biological activity, in the context of metabolic activity **(****Fig. 3****).**

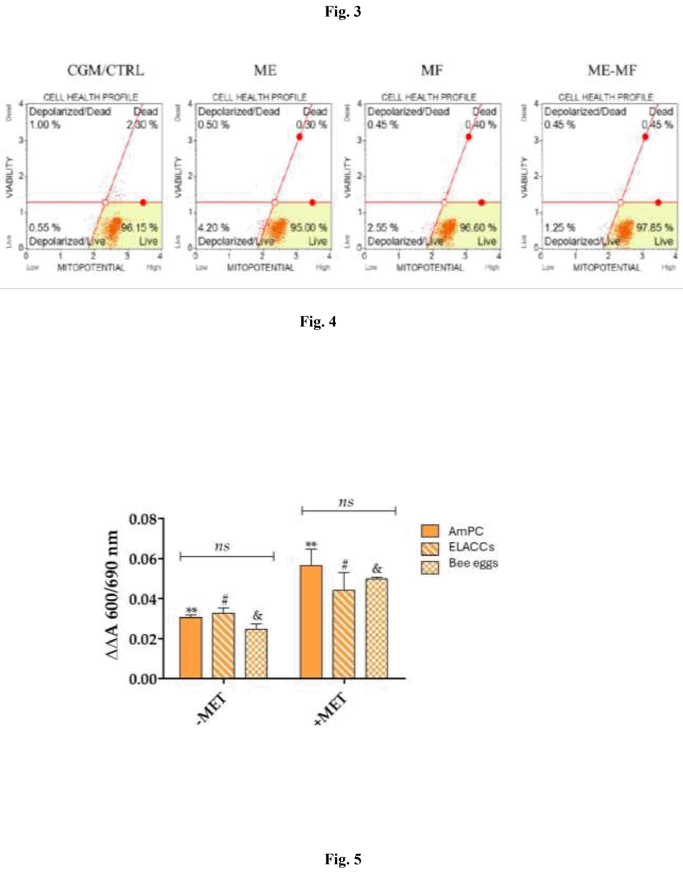

Evaluation of the effects of the tested compounds on the metabolic activity of cells isolated from bee eggs, expressed in terms of mitochondrial membrane potential, showed that heptakis(2,3,6-tris-O- methyl)cyclodextrin could decrease cell metabolism, thereby negatively affecting cell viability **(****Fig. 4****).** There was no significant effect of metformin on mitochondrial metabolism, while metformin in complex with methylated cyclodextrin (Met-ME) significantly increased cell metabolic activity.

The obtained results show that the obtained Met-ME complexes significantly affect the metabolic activity of cells isolated from honey bee eggs by enhancing mitochondrial metabolism. At the same time, Met-ME does not significantly affect the proliferative activity of bee cells.

**II.C A** bee cell-based system was also developed for testing active substances away from the animal model (testing different test formats, selecting the optimal one).

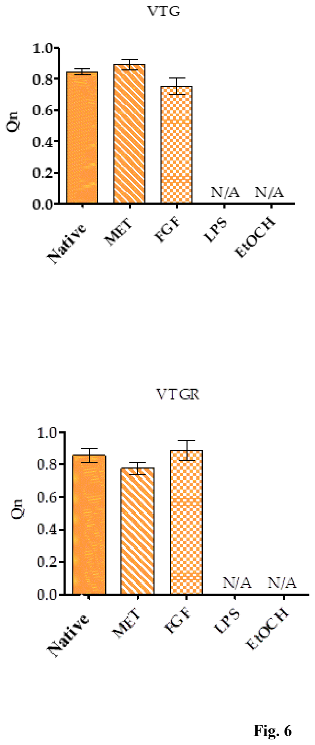

The model for testing active substances based on the use of honeybee material was chosen based on comparative analyses of the effect of the active substance (i.e. metformin at a concentration of 0.1 mM) on the metabolic activity of cells. The comparative studies included the effect of metformin on the metabolism of AmPC lines, ELACs as well as eggs. Evaluations of metabolic activity were carried out in 24-hour cultures, conducted in glucose-enhanced culture medium with 20% bovine serum and 2% antibiotic-antimycotic addition. The results indicated that the cellular response of the AmPC line, as well as ELACs, to the tested active substance converged. A similar biological effect, associated with an increase in metabolic activity, was observed in cultures in which eggs were maintained **(****Fig. 5****).** Hence, it was decided to continue studying the activity of active substances using bee eggs. The developed system is stable, gives reproducible results, and, more importantly, allows securing the selected material, providing constant access to cells with high viability, and thus continuity of material for research. Further studies have shown that a molecular marker that closely correlates with the viability of bee eggs in *in vitro* cultures is the expression level of mRNA for vitellogenin (VTG) and its receptor (VTGR). Studies of the effects of active substances, metformin, FGF as well as stress compounds LPS and EtOH indicate a close correlation of the metabolic activity of eggs with the expression of VTG and VTGR markers **(****Fig. 6****).**

### Example III - Effect of metformin and Met-CD and Met-ME complexes on the transcriptional activity of genes crucial to cell survival, as well as genes responsible for the pluripotent nature of progenitor cells isolated from honeybee eggs - in vitro tests - on cells

The aim of the study was to evaluate the transcriptional activity of genes crucial to cell survival, as well as genes responsible for the pluripotent nature of progenitor cells isolated from honeybee eggs.

### Materials and methods

A detailed description of the experimental cultures is included in Example II. After the *in vitro* model experiments were completed, the cultures were washed twice with Hanks' salt free of magnesium and calcium ions (HBSS) to remove residual culture medium. Each step of washing the cultures in HBBS included centrifugation of the eggs at room temperature for 3 minutes at 300×g. After centrifugation, the supernatant was removed. After the final washing step, cells were homogenized using 1 ml of Tri^{®} Reagent, Sigma Aldrich. Homogenization was carried out at room temperature for about 7 minutes. The slides were then frozen at - 80°C and stored until isolation of total RNA. Evaluation of gene expression was carried out using RT-qPCR technique involving the following steps:

### Isolation of total RNA by phenol-chloroform method

The thawed preparations were mixed thoroughly on a shaker for 1 minute. Then 200 µl of chloroform was added to the samples, and the samples were mixed by shaking for about 10 seconds. The samples were then left at room temperature for 7 minutes. After this time, the samples were centrifuged for 15 minutes (12,000×g/temperature 4°C). Total RNA was precipitated from the aqueous phase obtained after centrifugation of the slides. The aqueous phase was collected from each sample and transferred to a new eppendorf tube, and 1 µl of glycogen (UltraPure^{™} Glycogen, Life Technologies) was added. Then, isopropanol was added at a volume of 600 µl for every 1 ml of trizol used in the homogenization step. Samples were incubated for 7 minutes at room temperature, then centrifuged for 1 hour (12,000×g/temperature 4°C). The total RNA pellet was washed twice using 1 ml of 70% ethanol (Merck/Sigma Aldrich). During RNA washing, the following centrifugation parameters were used 7,500×g for 5 minutes at 4°C. Total RNA was dissolved with 30 µl of nuclease-free molecular biology water treated with diethylpyrocarbonate (DEPC); (Merck/Sigma Aldrich). Quantitative and qualitative analysis of the resulting RNA was performed at wavelength using a microplate reader (Epoch^{™} Microplate Spectrophotometer, BioTek).

### Synthesis of complementary DNA (cDNA)

Synthesis of cDNA was preceded by digestion of residual genomic DNA. The reaction was carried out using the Precision DNase kit (Primer Design, Blirt DNA Gdansk). Each reaction was performed using 1 µg of total RNA. The reaction involved digestion of residual gDNA in the preparations for 10 minutes at 30°C, followed by enzyme inactivation carried out at 55°C for 5 minutes. The gDNA-free RNA preparations were used for the reverse transcription reaction, which was performed using the Tetro cDNA Synthesis Kit (Bioline, Blirt DNA Gdansk). The reaction was performed in a total volume of 40 µl using oligo(dT)18 probes according to the manufacturer's recommendations. The reaction protocol included incubation of samples at 45°C for 30 minutes, followed by enzyme inactivation at 85°C for 5 minutes. The reaction was carried out in a T100 thermocycler (Thermo Cycler, BioRad).

### Preamplification of arrays

The preamplification reaction was carried out in a total volume of 40 µl. The reaction mixture consisted of cDNA in a volume of 4 µl, specific genetic probes (Table 1), which had a final concentration of 0.25 nM, and a 2-fold concentration of SensiFAST^{®} SYBR & Fluorescein (Bioline, Blirt DNA Gdansk). The reaction was carried out in a T100 thermocycler (Thermo Cycler, BioRad). The reaction protocol included (i) pre-denaturation at 95°C carried out for 2 minutes; (b) 18 repeating cycles, each consisting of denaturation (95°C/5 sec), primer attachment (62.2°C/3 min) and elongation of reaction products (72°C/5 sec). After completion of the reaction, the matrices were diluted 4-fold, frozen and stored for further analysis at -20°C.

**Table 1 Primer sequences used in the real-time PCR reaction**

| **Name of primer** | **Sequence 5'-3'** |
|---|---|
| Am-Oct4A-F | AAGCTCGGCTTCACCCAGG |
| Am-Oct4A-R | AACCTGCATATCGTCGTCTGG |
| Am-Oct4B-F | ACATGTGCAAGTTGAAGCCG |
| Am-Oct4B-R | GGTTTCGGCTGTTTGTGGAA |
| Am-SOX2-F | ACTTCCCACCATCAATCCGC |
| Am-SOX2-R | GGGTTCTCTTGGGCCATCTT |
| Am-Nanog-F | TCAGGTGTACGAGTTGGAGC |
| Am-Nanog-R | TGGGTGCTGGTCAGTTTCAG |
| Am-β-actin-F | ATCCTGGAATCGCAGATAGAATG |
| Am-β-actin-R | GGATGGTCCAGACTCGTCGTAT |
| Am-caspl-F | ACTGATGGTCAACCAGCTTCT |
| Am-caspl-R | CCGCGAGTGGTATTTCTCCA |
| Am-casp3-F | ATCACAACGAAACGGGACGA |
| Am-casp3-R | ATGCAGTCGCAATCATCGTG |
| Am-casp9-F | GCTTTTCCAGAAACAGTTCCAGT |
| Am-casp9-R | TCCACGAGGATTTCTACGCAT |
| Am-cytC-F | GCATCTATCCACGGTCAGGC |
| Am-cytC-R | GATCACCCGCAGGAATACCC |
| Am-buffy-F | AATTCTGGGAATGCAGGCCA |
| Am-buffy-R | TTCTCCGAGCCGAATCAACT |
| Am-lamin-F | CCATATTAAGTGGGCGAGCG |
| Am-lamin-R | AGTGCTTTTCTGTATCTTTTCAAGT |
| Am-p53-F | TGGAATATGACCACTACGAATGGA |
| Am-p53-R | AGGGTACAAATCCAATCCTTCAC |

### Analysis of the number of transcripts using real-time quantitative PCR (quantitative polymerase chain reaction (qPCR))

For the qPCR reaction, 1 µL of each of the matrices obtained at the preamplification step was used. The concentration of specific oligonucletotides in the reaction mixture was 0.5 µM. The reaction was carried out in a final volume of 10 µl using the SensiFAST^{®} SYBR & Fluorescein kit (Bioline, Blirt DNA Gdansk). Analysis was performed using the CFX Connect TM Real-Time PCR Detection System platform (BioRad^{®}). The reaction protocol included (i) pre-denaturation conducted at 95°C for 2 minutes; (ii) 40 cycles consisting of denaturation (95°C/5 sec), primer hybridization (62.2°C/10 sec) and elongation (72°C/5 sec).

Amplicon dissociation curve analysis was performed to determine the specificity of the PCR products. Melting curves were generated by raising the reaction temperature from 65°C to 95°C at a heating rate of 0.2°C/s and continuing to measure fluorescence. The specificity of the obtained products was also verified against NTC *(no template control)* and NRT *(no reverse transcriptase control).* The relative number of transcripts was calculated by the 2^{(-)(ΔΔ) (CT)}method using normalization to the sample with the lowest expression of the gene under study (_{RQMAX}). In formula used the following calculations: $ΔCT \left(reference sample\right) = CT of test gene - CT of reference gene .$ RQMAX= ΔCT (recorded in sample with lowest expression/maximum ΔCT) - ΔCT (test sample). β-actin was chosen as the reference gene.

### Results

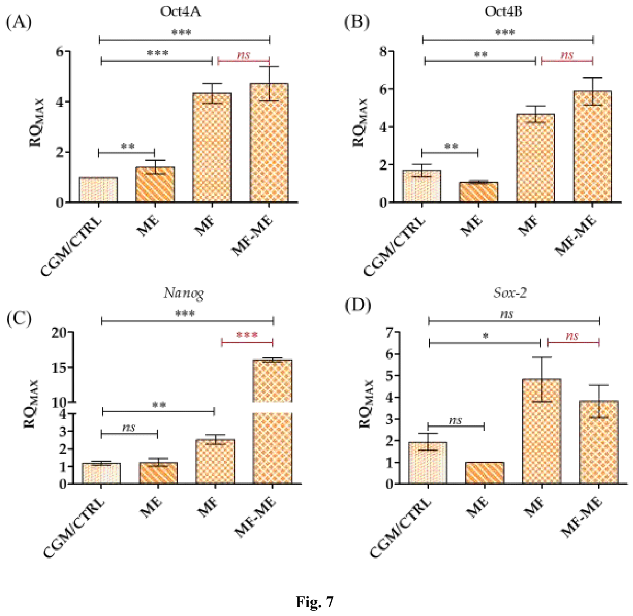

Comparative analysis of transcript levels showed a significant increase in Oct4A gene expression in all experimental cultures relative to the control culture (CGM/CTRL). For the Oct4B isoform, an increase in expression was noted in cells treated with pure metfromin and metformin contained in a complex with heptakis(2,3,6-tris-O-methyl)cyclodextrin. Both isoforms of the Oct4A gene are crucial in maintaining the progenitor nature of the cells. The Oct4A isoform is expressed in embryonic-like (pluripotent) cells, while expression of the Oct4B factor conditions cell proliferation, is also noted in progenitor lines, but with lower phenotypic plasticity (multipotent cells). This gene expression profile may indicate the presence of progenitor cells with different degrees of differentiation. In cells treated with heptakis(2,3,6- tris-O-methyl)cyclodextrin, Oct4A expression was higher than in control cultures, while Oct4B expression was significantly decreased. In this case, the significant decrease in the level of mRNA for Oct4B, may be related to the cytotoxic effect of ME, resulting in a decrease in metabolic and proliferative activity in the cultures. High levels of Oct4 gene expression in cells treated with Met and Met-ME correlated with increased levels of Sox-2 and *Nanog* gene expression. Such an expression profile is crucial for maintaining the pluripotent nature of progenitor cells - high mRNA levels of all transcripts determine the maintenance of an undifferentiated cell phenotype. The results show that the inclusion of Met in complex with ME, does not affect the levels of Oct-4A, Oct4B and Sox-2. Met- ME significantly increases the expression of *Nanog* factor **(****Fig. 7****).** Since *Nanog* is important in maintaining biochemical homeostasis of stem cells, this result may correlate with the increase in metabolic activity noted in these cultures.

### Expression analysis of genes involved in the process of apoptosis

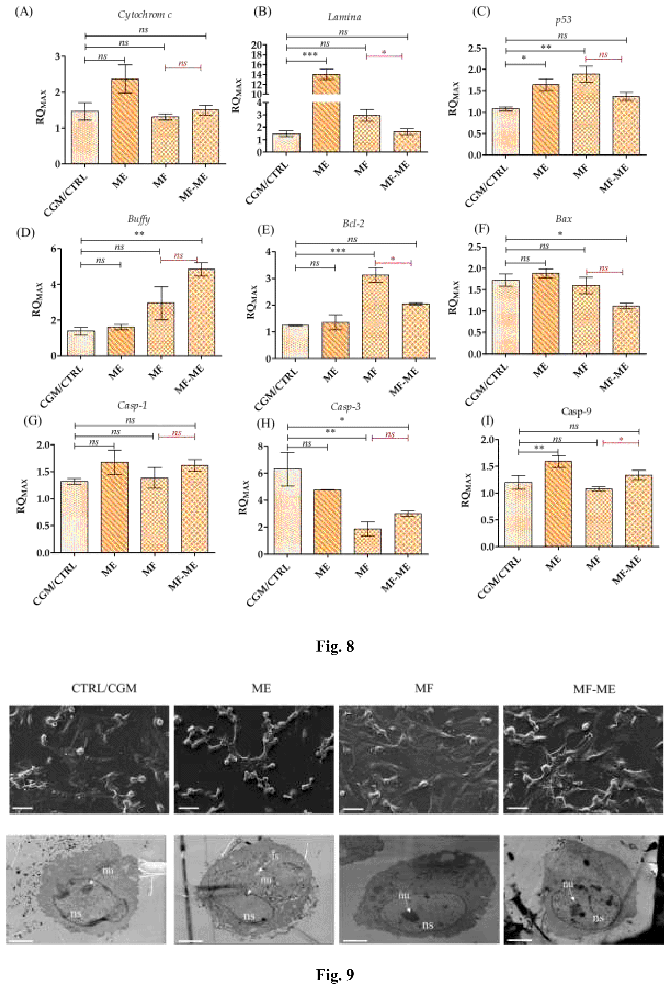

Evaluation of the expression profile of genes involved in programmed cell death proves the anti-apoptotic effect of metformin, as well as metformin in complex with heptakis(2,3,6-tris- O-methyl)cyclodextrin **(****Fig. 8****).** Cultures treated with Met and Met-ME showed decreased expression of pro-apoptotic genes such as *Bax and Casp-3* and increased transcript levels of anti- apoptotic genes, i.e. *Bcl-2* and *Buffy.* There were no significant differences between control and Met and Met-ME cultures for the mRNA levels of genes encoding the expression of *cytochrome c, lamina, caspase-1* and *caspase-9.* The expression levels of these genes increase during apoptosis, stable expression of these transcripts means that they are not activated in Met and Met-ME treated cultures. In cultures treated with Met and ME, there was an increase in mRNA expression for *p53,* while in cultures treated with Met-ME, the level of this transcript was similar to that observed in the control culture. In the case of ME-treated cells, high levels of p53 correlate with high expression of genes associated with apoptosis induction, such as *cytochrome C, lamina* and *caspase 9.* This result correlates with the reduced cell viability determined in these cultures. Differences in the expression of genes associated with programmed cell death noted between Met and Met-ME cultures were related to the mRNA levels of genes encoding *lamina, caspase 9* and *Bcl-2.* In Met cultures, elevated expression of lamina may correlate with increased expression of *p53,* while in the Met cultures recorded higher expression of the anti-apoptotic gene *Bcl-2* and reduced expression of *caspase.*

### Effect of studied factors on cell ultrastructure

The study was carried out using a scanning microscope as well as a transmission microscope. In addition, analysis was carried out using an epifluorescence microscope visualizing live (calcein stained) and dead (propidium iodide stained) cells. Analysis of the morphology of cells cultured under control conditions showed that cells isolated from eggs *in vitro* are a heterogeneous population - in addition to the typical embryo-like clusters of cells, spindle-shaped cells are also visible in the microscopic image. In cultures treated with heptakis(2,3,6-tris-O-methyl)cyclodextrin, fibroblast-like (spindle-like) cells were shrunken. In contrast, in cultures in which metformin was added, spindle-like cells dominated, forming areas of high density (confluence). In cultures where Met was administered in complex with ME, the cultures were also characterized by greater confluence, but with distinct aggregates of spherical (embryo-like) cells.

Ultrastructure analysis showed no significant changes in cellular organization, in cultures treated with Met and Met-ME **(****Fig. 9****).** The cells were characterized by a peripherally (peripherally) located euchromatic cell nucleus *(ns),* in which the nucleolus *(ne)* was marked. In cultures treated with ME, there was an increase in the presence of lysosomal vesicles (*ls*) and a less marked area of the nucleus.

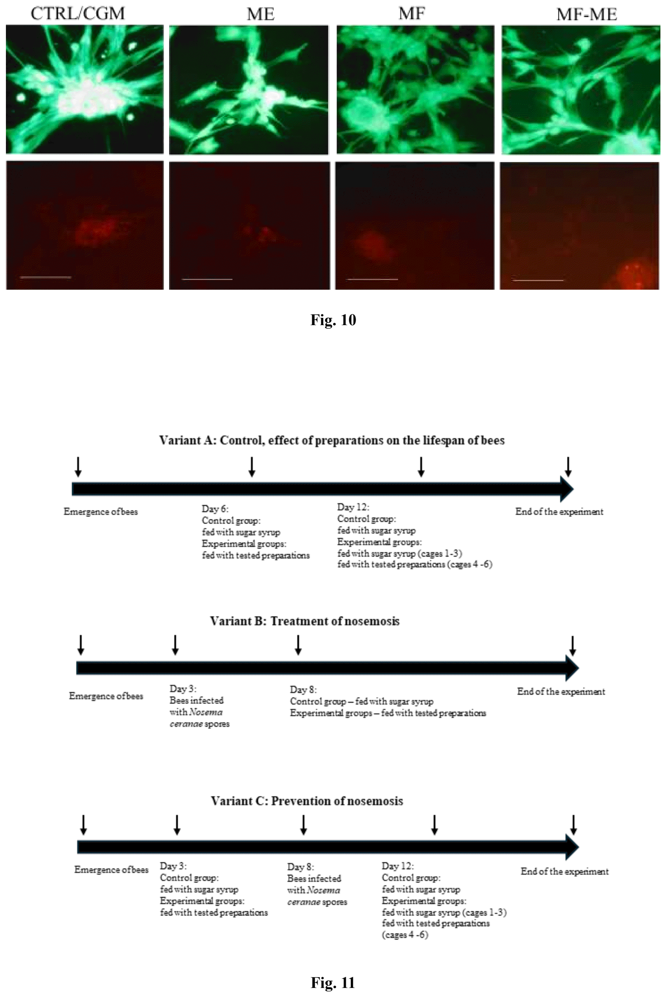

Analysis of cultures stained with propidium iodide, showed an increase in the percentage of dead cells in areas of culture confluence **(****Fig. 10****).** Based on the images obtained by epifluorescence microscopy, it is also possible to infer the effect of the studied factors on cell confluence. These images confirm the observations made using electron microscopy and indicate the cytotoxicity of ME. The results of microscopic analyses are also consistent with the results of cell viability and metabolic activity analyses, as an increase in cell confluence was observed in cultures treated with Met and Met-ME, which is closely related to increased cell metabolism and proliferative activity. A sensitivity of cells to calcein was also observed, which was associated with cell detachment from the culture medium just after staining, due to this, for assessing the viability of cultures, Annexin V/propidium iodide staining and cytometric analysis seems to be a better test.

### Summary

The results show that the obtained metformin complexes with heptakis(2,3,6-tris-O- methyl)cyclodextrin have anti-apoptotic activity and modulate phenotypic plasticity of progenitor cells. Based on the determined gene expression profiles, it can be concluded that the biological activity of metformin, or metformin in Met-ME complex, is similar. Both compounds have a significant effect on stabilizing the phenotype of progenitor cells. However, metformin in combination with cyclodextrin significantly elevated *Nanog* gene expression, which may also be key in the context of increased viability of these cultures. The expression profile of apoptosis- related genes and microscopic images confirm previous results of metabolic assays, which showed that both Met and Met-ME positively affect the viability and metabolism of progenitor cells isolated from bee eggs.

### Example IV - Effects of metformin and Met-CD complexes on the expression of bee immunity-related genes and the control and prevention of nosema - in vivo tests - on bees - cage tests

### Research Methodology

### Effect of preparation on honey bee nosema

The tests were conducted in accordance with the methodology of OECD/OCDE 214, dated 21.09.1998, "OECD GUIDELINES FOR THE TESTING OF CHEMICALS; Honeybees, Acute Oral Toxicity Test." In the experiment, 18 food groups were formed and an additional 6 food groups were separated in the second half of the experiment. Each food group consisted of 6 cages, in which 40 *Apis mellifera* workers of the same age (from winnowing) were kept. In summary, the experiments were conducted on 25 basic food groups with 240 bees in each group The experiments in total were conducted on 150 cages with 6,000 bees.

### Laboratory tests

The scheme of the experiments carried out is presented in Fig. 11. In the experiment, the effect of the preparation on honey bee nosema. The following food groups were established:
1A (control - eradication), 1B (control - prevention), 1C (healthy bees - control) 2A (0.1 mM - eradication),
2B (0.1 mM - prevention), 2C (0.1 mM - healthy bees) 3A (0.5 mM - eradication), 3B (0.5 mM - prevention),
3C (0.5 mM - healthy bees)

### Variant A: Healthy bees - 6 cages each

Control - healthy bees. In this group, the bees were not infected and were kept as a control to determine the life span of the bees after administration. Fumagilin-B, which contains fumagillin as an active ingredient (antibiotic), was used as a positive control. On the twelfth day of the experiment, the food groups were further divided. Bees in the food groups were fed preparations and later sugar syrup **(****Fig.11****,** food group sugar syrup 1 - 3), while bees in the food groups were fed sugar syrup enriched with preparations throughout the experiment **(****Fig. 11****,** food group preparation according to the invention 4 - 6).

### Variant B: Nosema control - 6 frames each.

Control of nosema. In this group, bees were first infected with material containing *N. ceranae* spores and later administered. Fumagilin-B was used as a positive control.

### Variant C: Prevention of nosema - 6 frames each

Prevention of nosema. In this group, the bees were first treated and then infected with material containing N. *ceranae* spores. Fumagilin-B was used as a positive control. On the twelfth day of the experiment the food groups were further divided. The bees in the food groups were fed preparations and later sugar syrup, while the bees in the food groups were fed sugar syrup enriched with preparations throughout the experiment.

### Expression of immune genes

RNA isolation was performed from 3 honey bee abdomens using the Universal Purficiation Kit (EURx, Gdansk, Poland). The isolated RNA was purified from residual DNA using TURBO DNA-free^{®} (ThermoFischer Scientific, Waltham, MA, USA). RNA concentration was determined in the purified material (absorbance measurement at λ = 260 nm) and the purity of the obtained RNA was checked (measurement ratio at two wavelengths 260/280 nm). RNA samples were reverse transcribed using a cDNA reverse transcription kit with RNase inhibitor (ThermoFischer Scientific, Waltham, MA, USA). The number of transcripts was normalized against the β-actin reference gene.

### Results

### Option A: Healthy Bees

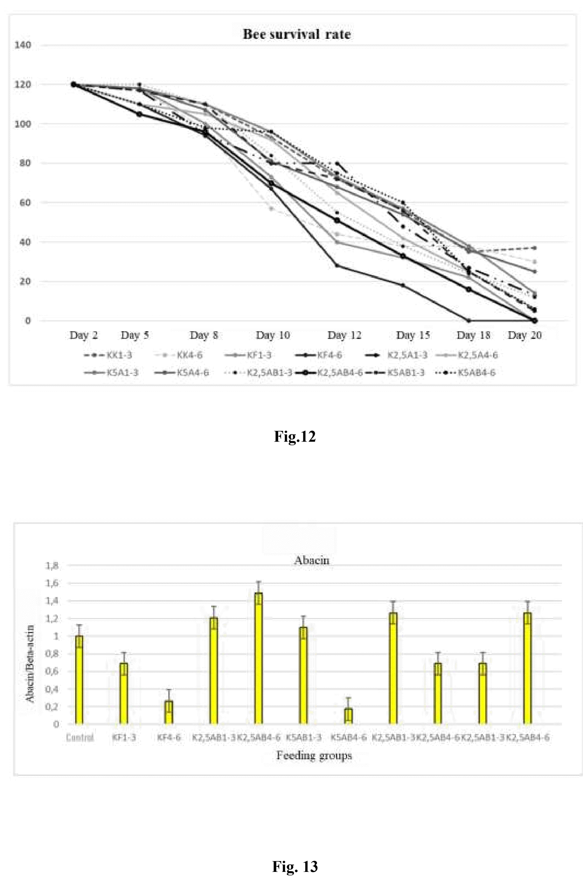

In all groups, bees took the preparations equally readily. Bee survival rates in the A0.1 (Met 0.1 mM), A0.5 (Met 0.5 mM) and AB0.1 (Met-CD 0.1 mM), AB0.5 (Met-CD 0.5 mM) food groups were higher than in the control group **(****Fig. 12****).** A clear toxic effect can be observed for the group fed sugar syrup with fumagillin, which clearly increased bee mortality.

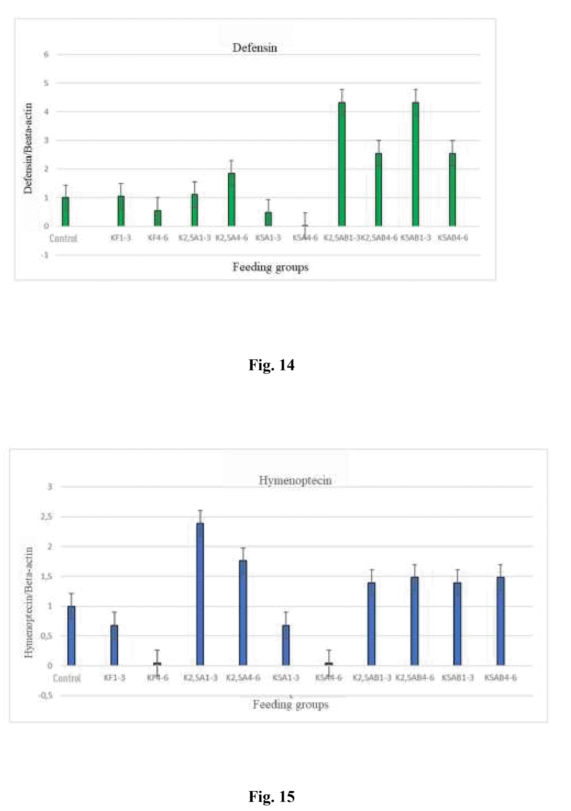

The expression level of immunity-related genes in the honeybee is shown in **Figs. 13****,** **14 and 15****.** The designations of the preparations used in **Figs. 13****,** **14** and **15** include 1 - 3 indicating food groups 1 - 3 (sugar syrup) in **Fig. 11** and 1 - 4 indicating preparation according to the invention 4 - 6 in **Fig. 11** (e.g., K2.5A4-6 indicates that preparation according to the invention was administered). Groups in which fumagillin was administered had markedly reduced expression of the immune genes tested, especially abecin and hymenoptecin. Administration increased abecin expression, especially the K2.5A4-6 (Met 2.5mM) and K2.5AB4-6 (Met-CD 2.5 mM) groups, indicating that longer administration has a positive effect on abecin expression **(****Fig. 13****).** In the case of defensin, a marked increase in K2.5AB1-3 (Met-CD 2.5 mM), K2.5AB4-6 (Met-CD 2.5 mM), K5AB1-3 (Met-CD 5.0 mM) and K5AB4-6 (Met-CD 5.0 mM) **(****Fig. 14****).** For hymenoptecy, a strong increase in expression was noted in the K2.5A1-3 (Met 2.5 mM) and K2.5A4-6 (Met 2.5 mM) groups **(****Fig. 15****).**

### Option B: Combating nosema.

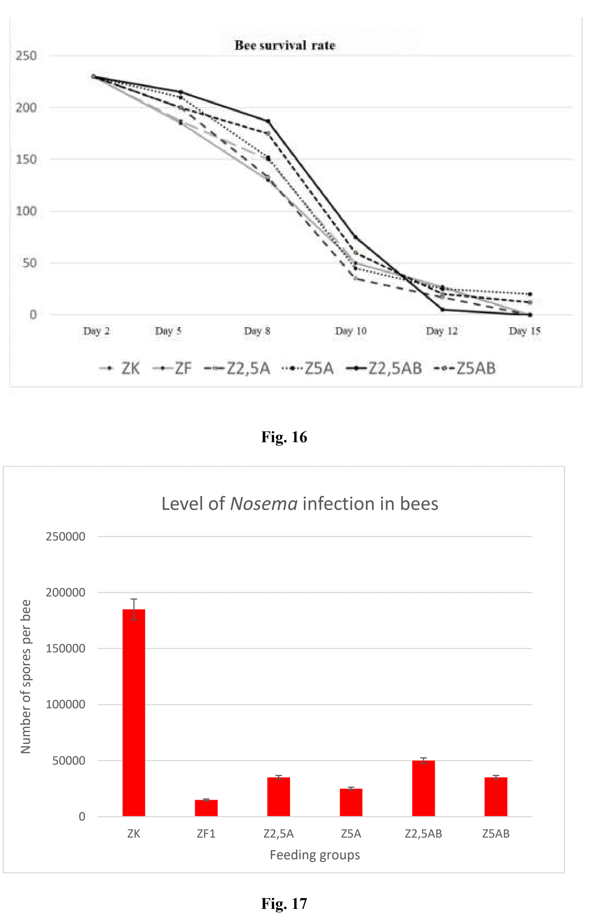

In all groups, the bees took the preparations equally readily and in similar amounts. The group was infected with nosema and took slightly more food compared to the control (variant A), which is related to the higher energy requirements of the bees during the development of the infection. Bee survival in all food groups was higher than in the control group, which included infected bees and fed only sugar syrup **(****Fig. 16****).**

In Variant B: nosema control, a decrease in the level of infection was observed in the groups of bees in which the preparations were administered **(****Fig. 17****).** The most effective was the addition of fumagillin, which almost completely reduced the infection. Comparable efficacy was observed for preparations Z5A and Z5AB (Met-CD 5.0 mM).

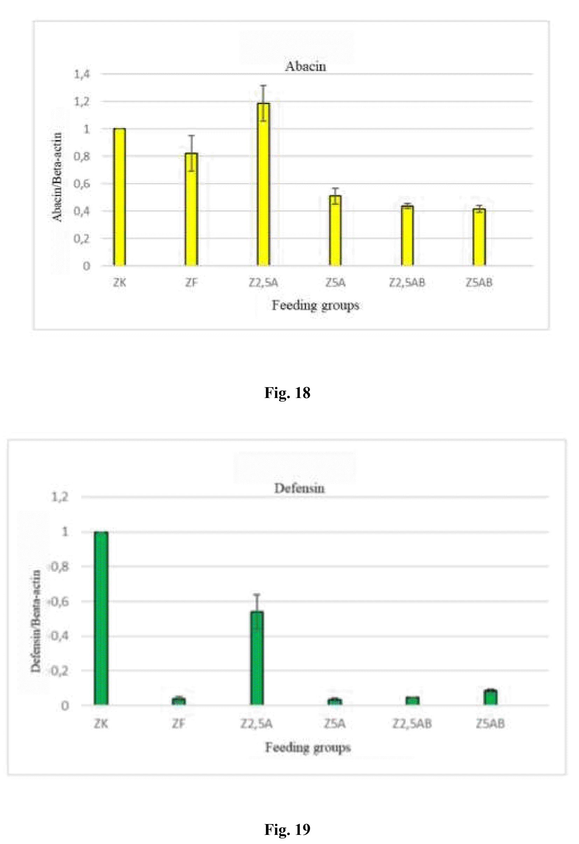

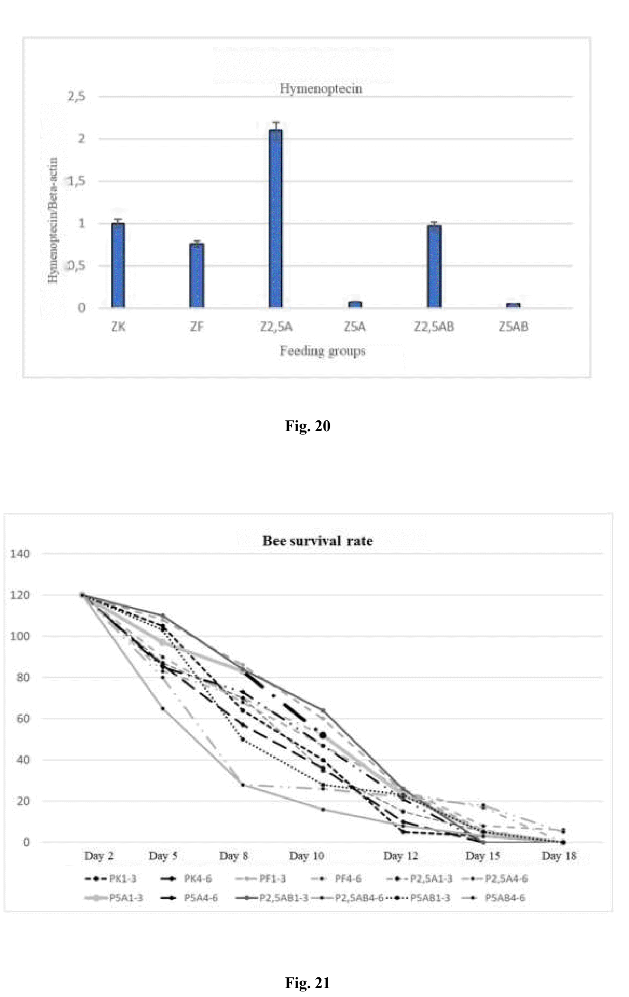

The expression level of immunity-related genes in the honeybee is shown in **Figs. 18, 19** **and** **20****.** Groups in which fumagillin was administered had reduced expression of the immune genes tested, especially defensin. The administration increased the expression of abecin in the Z2.5A group (Met 2.5 mM), while the other preparations decreased abecin expression **(****Fig. 18****).** For defensin, a marked decrease in immune gene expression is seen in all food groups, perhaps related to the therapeutic effect of the preparations (Fig. 19). For hymenoptecin, a strong increase in expression was seen in the Z2.5A (Met 2.5 mM) group, while there was a decrease for the Z5A (Met 5.0 mM) and Z5AB (Met-CD 5.0 mM) groups **(****Fig. 20****).**

### Option C: Prevention of nosema.

The group was first fed with food, then infected with nosema and then, depending on the subgroup, fed with either pure sugar syrup or sugar syrup enriched with preparations. The bees in the P2.5AB1-3 (Met-CD 2.5 mM), P2.5AB4-6 (Met-CD 2.5 mM), P5AB1-3 (Met-CD 5.0 mM) and P5AB4-6 (Met-CD 5.0 mM). Bee survival rates in all food groups were higher than in the control, which included infected bees and fed only sugar syrup **(****Fig. 21****).**

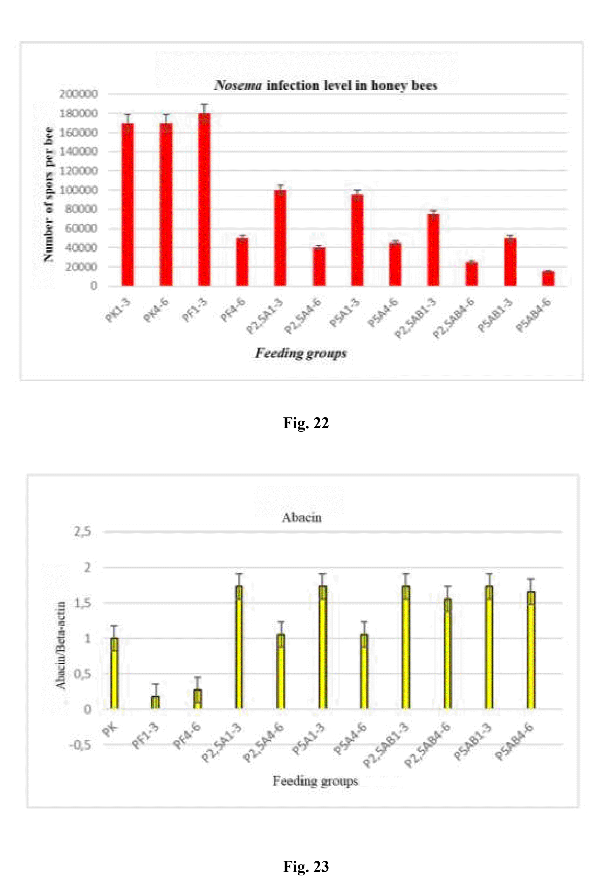

In Variant C: prevention of nosema, a decrease in the level of infection was observed in groups of bees in which all preparations except fumagillin from Variant 1 - 3 were administered **(****Fig. 22****).** The designations of the preparations used in **Fig. 22** include 1 - 3 indicating food groups 1 - 3 (sugar syrup) in **Fig. 11** and 1 - 4 indicating preparation according to the invention 4 - 6 in **Fig. 11** (e.g., P2.5AB4-6 indicates that preparation according to the invention was administered). As can be seen, it is pointless to administer an antibiotic (fumagillin) as a means of preventing the development of the disease. The administration of fumagillin after infection resulted in a decrease in infection. Administered preparations A and AB prevented the development of nosema and protected the bees from developing infection (groups: P2.5A1-3 (Met 2.5 mM), P5A1-3 (Met 5.0 mM, P2.5AB1-3 (Met- CD 2.5 mM), P5AB1-3 (Met-CD 5.0 mM)). Administration of the preparations after infection enhanced their therapeutic effect (groups: P2.5A4-6 (Met 2.5 mM, P5A4-6 (Met 5.0 mM), P2.5AB4-6 (Met-CD 2.5 mM), P5AB4-6 (Met-CD 5.0 mM)).

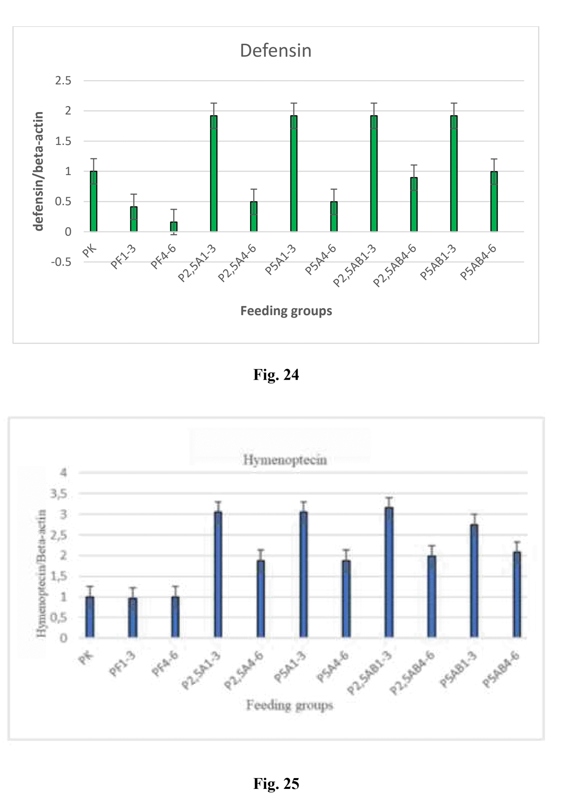

The expression level of immunity-related genes in the honeybee is shown in **Figs. 23****,** 24 and 25**.** The designations of the preparations used in Figs. **23****,** **14** and **15** include 1 - 3 indicating food groups 1 - 3 (sugar syrup) in **Fig. 11** and 1 - 4 indicating preparation according to the invention 4 - 6 in **Fig. 11** (e.g., K2.5A4-6 indicates that preparation according to the invention was administered). Groups in which fumagillin was administered had decreased expression of abecin and defensin. The administration increased abecin expression in the P2.5A1-3 (Met 2.5 mM), P5Al-3 (Met 5.0 mM), P2.5AB1-3 (Met-CD 2.5 mM), P2.5AB4-6 (Met-CD 2.5 mM) groups, P5AB1-3 (Met-CD 5.0 mM) and P5AB4-6 (Met-CD 5.0 mM) (Fig. 23). In the case of defensin Prolonged administration in groups 4 - 6 silenced the expression of this gene and an increase in expression levels was noted for groups P2.5A1-3 (Met 2.5 mM), P5A1-3 (Met 5.0 mM), P2.5AB1-3 (Met-CD 2.5 mM) and P5AB1-3 (Met-CD 5.0 mM) (Fig. 24). For hymenoptein, an increase in expression was noted for all A and AB groups (Fig. 25).

In variant C, in which the effectiveness of the preparations in protecting bees from infection was tested, a decrease in the level of infection was observed in groups of bees in which all preparations except fumagillin from variants 1 - 3 were administered. The administration of an antibiotic (fumagillin) as a means of preventing the development of the disease is unwarranted, while the administration of fumagillin after infection resulted in a decrease in infection. Administered preparations A and AB prevented the development of nosema and protected the bees from developing infection (groups: P2.5A1-3 (Met 2.5 mM), P5Al-3 (Met 5.0 mM), P2.5AB1-3 (Met-CD 2.5 mM), P5AB1-3 (Met-CD 5.0 mM)). Administration of the preparations after infection enhanced their therapeutic effect (groups: P2.5A4-6 (Met 2.5 mM), P5A4-6 (Met 5.0 mM), P2.5AB4-6 (Met-CD 2.5 mM), P5AB4-6 (Met- CD 5.0 mM)).

In the experiment conducted, fumagillin was administered in the form of a commercial preparation called Fumagilin-B, which is an agent for the control of nosema in honey bees. The mechanism of action of fumagillin is to inhibit the activity of the methionine aminopeptidase enzyme MetAP-2 (Van der Heever et al., 2014). Fumagilin-B contains an extract of the fungus *Aspergillus fumigatus* and until recently had highly specific activity in the prevention and treatment of nosema, with proven high efficacy against both *Nosema ceranae* and *N. apis.* However, recent studies have shown that fumagillin used against *N. ceranae* is not effective, as the pathogen has begun to develop resistance to the drug (Huanga et al., 2013). Fumagillin was withdrawn from the European Union in 2016 by the European Medicines Agency due to the permeation of fumagillin residues into honey and other bee products, which could be dangerous to consumers of honey and bee products (Regulation 2377/90, European Commission 2009; Van der Heever et al., 2014).

Depending on the level of infection and the condition of the bees, the expression of genes encoding immune peptides in *Nosema* spp. infected bees may be silenced (Antúnez et al., 2009) or, on the contrary, may be stimulated (Chaimanee et al., 2012, Iliasov et al., 2013). In the experiments conducted, a clear increase in the expression of the tested immune genes was observed for most of the variants, which indicates positive stimulation of the bee's immune system under the influence of the preparations and is important for strengthening the immune response of the honeybee (Biliková et al., 2001). This is a major advantage of the preparations over the action of antibiotics such as fumagillin, which have an immunosuppressive effect on bees (Miyagi et al., 2000).

In conclusion, the tested preparations at concentrations not exceeding 2.5 mM in cage tests and 5 mM on the apiary are safe for bees. They improve bee immunity and can be used both as prophylactic agents and to control honey bee nosema.

### Example V - Positive effect of metformin on bee health (reduction of bee pathogens) - in vivo tests - on bees - field tests

### Experimental apiaries

The experiments were conducted in the experimental apiary after the bee colonies were administered MetCD at a concentration of 2.5 mM. Preparations were administered to 16 bee colonies in the experimental apiary. The preparations were administered around the clock in two apiaries on 16 research colonies, including 8 strong and 8 weaker colonies. At the same time, 16 control colonies were observed, to which the preparation was not administered. The first administration of syrup with the preparation took place in the second half of September and lasted 48 hours in the fastest-taking families. The second time the preparation was administered was in the spring. During the period of administration of the preparation with syrup, maximum daytime temperatures reached about 15^{°} °C, but at night there were cooler temperatures in the range of 5 °C.
**Apiary** 1 (two administrations autumn - 2 × 5 L per family in sugar syrup 3:2) - spring two administrations
**Apiary** 2 (two administrations autumn - 2 × 5 L per family in sugar syrup 3:2) - spring two administrations
**Control** - the preparation was not administered

Bees from the apiaries were transported in thermoses filled with liquid nitrogen and with thermal inserts placed on top of the bees. After being transported to the laboratory, the bees were placed in a low-temperature freezer at -79°C. The bees collected from the apiaries were tested for pathogens such as deformed wing virus, black mated virus, acute bee paralysis virus, *Nosema apis and Nosema ceranae,* gregarines, hurmastes, trypanosomatida, bee otorrhea, larvae lase, and bee marmot.

### Isolation of total RNA from the obtained samples and PCR reactions to check the presence of RNA viruses

Total RNA was isolated using the FastRNA Green Kit (MP Biomedicals) according to the kit manufacturer's protocol (with minor modifications). For each sample, abdomen from 3 bees were collected and crushed. The preparations were homogenized in RNApro Solution using 1.4 mm diameter ceramic beads (Lysing Matrix D) and ¼ inch ceramic beads in a FastPrep-24 5G homogenizer (MP Biomedicals) (lysis parameters: time 40 s, speed 6 m/s, room temperature). The precipitation step using anhydrous ethanol was carried out for 3 h. The amount of total RNA preparations obtained was checked fluorimetrically in a Qubit 2.0 instrument using the Qubit RNA HS Assay Kit (Thermo Fisher Scientific). Total RNA was reverse transcribed (1.5 µg RNA) with random primers using the High-Capacity cDNA Reverse Transcription Kit (Thermo Fisher Scientific) according to the manufacturer's instructions. The resulting cDNA was then used as a template in a series of PCR reactions using ready-to-use PCR Mix Plus (A&A Biotechnology). The PCR reactions used 2 µl of cDNA each, and the final concentration of primers was 0.4 µM.

### Isolation of genomic DNA (gDNA) from the obtained samples and PCR reactions to check the presence of various bee pathogens

gDNA was isolated using the Tissue & Bacterial DNA Purification Kit (EURx) according to the kit manufacturer's protocol (with minor modifications). For each sample, abdomen from 3 bees were collected and crushed. The preparations were homogenized in Lyse T solution using 1.4 mm diameter ceramic beads (Lysing Matrix D) in a FastPrep-24 5G homogenizer (MP Biomedicals) (lysis parameters: time 40 s, speed 6 m/s, room temperature). The lysis step was carried out for 3 hours at 56°C in a thermomixer (500 rpm). The amount of gDNA preparations obtained was checked fluorimetrically in a Qubit 2.0 instrument using the Qubit dsDNA BR Assay Kit (Thermo Fisher Scientific). The quality of the gDNA preparations was checked by agarose electrophoresis, which showed a high degree of integrity of the gDNA isolates. The obtained gDNA was then used as a matrix in a series of PCR reactions using ready-to-use PCR Mix Plus (A&A Biotechnology). The PCR reactions used 100 ng of gDNA each, and the final concentration of primers was 0.4 µM.

### PCR reactions for deformed wing virus (DWV):

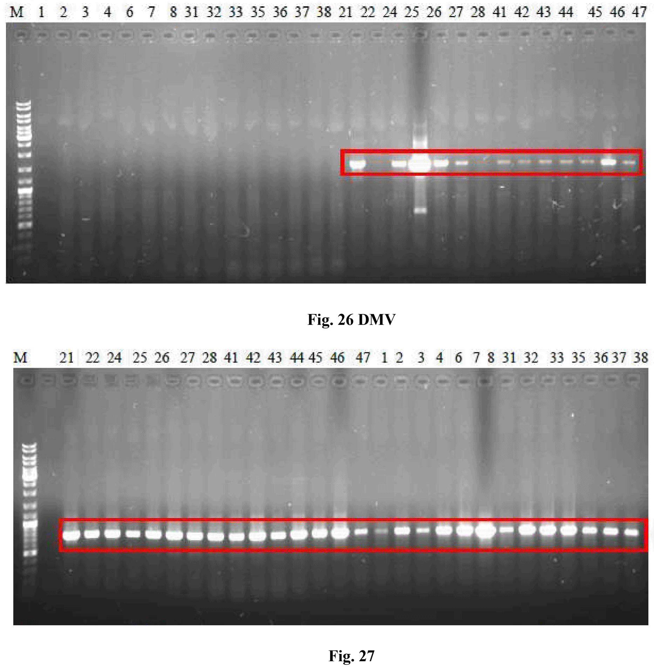

The PCR reaction series used primers Fstd (5'- GGACCATCCTTCCAGTCTACGAT-3') and Bstd (5'-CTGTAGGTTGTGCTCCTGATGAAGA-3') amplifying a 1,520 pz fragment from DMV virus cDNA (Gisder et al., 2009). PCR products **(****Fig. 26****)** were obtained on cDNA arrays prepared from samples from the control group (21 - 47), while the desired fragment size was not amplified on cDNA arrays from the test group (1 - 38).

### PCR reactions for black queen cell disease virus (BQCV):

The PCR reaction series used primers BQCV1 (5'- TGGTCAGCTCCCACTACCTTAAAC-3') i BQCV2 (5'-GCAACAAGAAGAAACGTAAACCACI-3') amplifying a 700 pz fragment from BQCV virus cDNA (Benjeddou et al., 2001). The desired length of PCR products **(****Fig. 27****)** was obtained on the templates of all prepared cDNA preparations - from both control and test group samples. However, to note, a large lower amount of genetic material for BQCV was observed in samples 1, 3, 31, 36, 38 **(****Fig. 27****)**

### Multiplex PCR reactions for parasitic protozoa from the orders of Trypanosomatida and Neogregarinorida

A series of multiplex PCR reactions used primers SEF (5'- CTTTTGGTCGGTGGAGTGAT-3') and SER (5'- GGACGTAATCGGCACAGTTT-3') enabling detection for *N. apis,* as well as NeoF (5'-CCAGCATGGAATAACATGTAAGG-3') and NeoR (5'-GACAGCTTCCAATCTCTAGTCG-3'), amplifying fragments of 420 pz *(Trypanosomatida* order) and 260 pz *(Neogregarinorida* order), respectively, from the gDNA of parasitic protozoa (Meeus et al., 2010).

In a series of multiplex PCR reactions **(****Fig. 28****),** PCR products of 260 pz, enabling detection of parasites of the order *Trypanosomatida,* were obtained on the matrices of most of the prepared gDNA preparations - both from samples from the control group and from samples from the test group. On the other hand, a fragment of 420 pz, enabling detection of parasites of the order *Neogregarinorida,* was obtained on gDNA arrays isolated from 3 samples (24, 27 and 43) from the control group.

### PCR reactions for the bee hornworm fungus (Ascosphaera apis):

The PCR reaction series used primers 3-F1 (5'-TGTCTGTGCGGCTAGGTG-3') and 3- R1 (5'-CCACTAGAAGTAAATGATGGTTAGA-3') allowing amplification of a fragment of approx. 400 pz specific for *A. apis* (James and Skinner, 2005). In a series of multiplex PCR reactions **(****Fig. 29****),** the desired length PCR products were obtained on the matrices of most of the prepared gDNA preparations - both from samples from the control group and from samples from the test group (except samples 3 and 4).

### Reactions PCR for the presence of spore-forming bacteria Paenibacillus larvae (larvae lasso):

A series of PCR reactions used primers Primer 1 (5'- AAGTCGAGCGGACCTTGTGTTTC-3') and Primer 2 (5'- GGAGACTGGCCAAAACTCTATCT-3') allowing amplification of a fragment approximately 1,000 pz long, specific for *P. larvae* (Govan et al., 1999). In a series of PCR reactions **(****Fig. 30****),** the desired length PCR products were obtained on the matrices of most of the prepared gDNA preparations, both from samples from the control group and from samples from the test group.

### Summary

The results of genetic tests indicate that the observed reduction in the number of such pathogens as *Trypanosomatida* and *Neogregarinorida, Ascosphaera apis, Paenibacillus larvae* and the tested wisps (*DWV and BQCV*) in bee colonies where a double dose of the preparation was administered (samples 1 - 4 and 31, 32, 35 and 36). Genetic test results were also administered *to Bombyx mori L., Antheraea pernyi,* and *Bombus lucorum* also obtaining identical agent effects to the honeybee.

### Example VI - Effects of metformin on the bee microbiome - in vivo tests - on the bees - field tests

### Apiary tests

The preparations were administered in the apiary in a single dose, which was taken up by the bees on the same day. The weather on the day the preparations were administered was nice, the sky with light cloud cover, the temperature was 28°C. Three bee colonies were separated from the apiary. One colony was designated as a control, the second colony was given preparation A, while the third colony was given preparation AB (Met-CD).

### Preparation of preparations

Preparation A: 157.5 mg of substance A dissolved in 500 ml of sugar syrup.
AB preparation: 1,575 mg of AB substance dissolved in 500 ml of sugar syrup.

The preparations were administered in the apiary in a single dose, which was taken up by the bees on the same day.

Preparation of 5 mM A: 157.5 mg of substance A dissolved in 500 ml of sugar syrup. 5 mM AB preparation:
1,575 mg of substance AB dissolved in 500 ml of sugar syrup.

Bees from all three colonies showed a low level of nosema infestation characteristic of this time of year. The control colony and the research groups were infested at a rate of 200,000 spores per bee on the day of application. Infection at a similar level (about 200,000 spores per bee) was also recorded one week and two weeks after the preparations were administered. Despite the persistence of light nosema infestation, the condition of bee colonies administered with both preparations A and AB was better assessed by the beekeeper. The colonies also showed an increase in honey productivity reaching 15% in the colony given Preparation A and 10% for the colony given Preparation AB.

The expression levels of immunity-related genes in the honey bee are shown in **Figs. 31, 32 and 33****.** Feeding preparations on the apiary increased the expression of abecin **(****Fig. 31****)** and defensin **(****Fig. 32****).** For hymenoptecin, an increase in expression was noted for the colony fed with preparation A **(****Fig. 33****).**

### Example VII - Determination of bee-safe doses of metformin and the complex met-CD- in vitro tests - on bees - cage and field tests

### Cage experiments administration of Met25 and Met100 to bees in cage experiments

The bees were divided into 3 food groups. Each food group consisted of 6 cages with 40 *Apis mellifera* individuals in each. In summary, each group consisted of 240 bees and a total of 18 standard cages with 720 bees were used in experiment one. The bees were fed preparations at concentrations of 25 mM (Met25) and 100 mM (Met100) dissolved in 1:1 w/v aqueous sugar syrup. The experiment was conducted to the last live bee in the following food groups:

### Research groups:

1. Control - sugar syrup - 6 cages (240 bees),
2. Met25-enriched food throughout the experiment - 6 cages (240 bees),
3. Met100 enriched food throughout the experiment - 6 cages (240 bees).

The bees had free access to the food. The designated food groups were the control, which included bees fed aqueous sugar syrup v/w 1:1 *ad libitum* and bees fed Met25 and Met100 preparations.

### Results

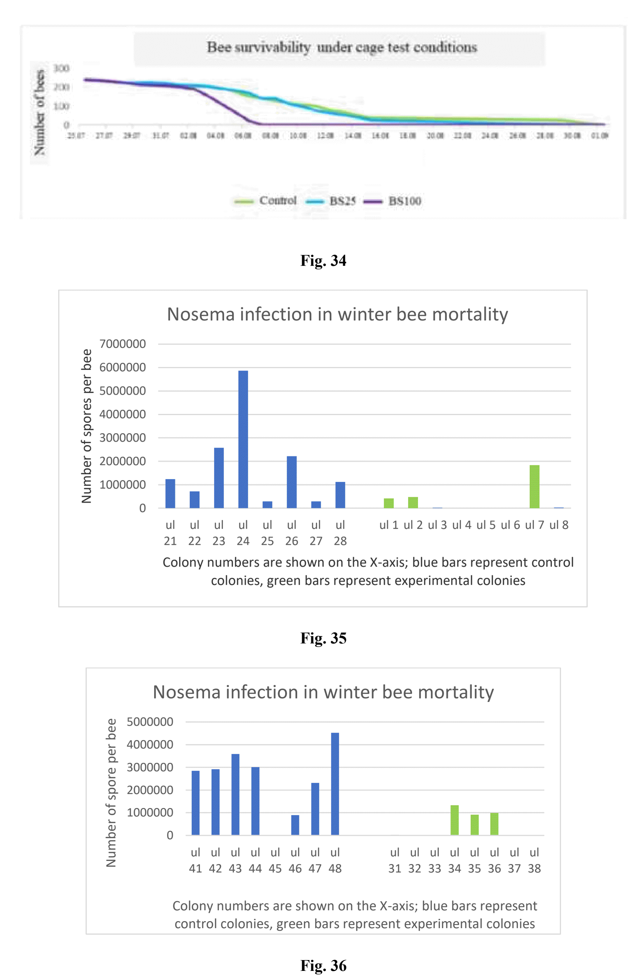

One-day-old honey bees were manually inoculated into cages on July 25, with 40 individuals per cage. The bees were observed until the last live bee, i.e., until the 38th day of the experiment (July 25 - September 01). Research schedule and analysis of results:
25.07 - Seeding of bees into cages, feeding young bees with sugar syrup, in accordance with EU OECD standards for conducting experiments on live bees
27.07 - feeding Met25 and Met100 in sugar syrup
02.08 - Increased mortality observed in Met100 group
06.08 - all bees in the Met100 group died (on the 10th day after Met100 was administered)
29.08 - all bees of the Met25 group died
01.09 - the last control bees died
The survival rate of bees in the study groups is shown in Fig. 34.

### Summary

There was increased mortality in the Metl00 group, while bee mortality in the Met25 group was comparable to the control, so Met25 can be considered a safe dose for bees.

### Food intake

The highest food intake was recorded in the control group. In the Met25 group, food intake was 74% relative to the control. In the Met100 group, food intake was 42% relative to the control. Food intake in the Met25 group was comparable to the control, where the bees were fed pure sugar syrup. In contrast, the Met100-enriched food was taken up by the bees at a significantly lower rate.

### Summary

Analysis of cage tests indicates that the safe dose of the product is Met25. The Met100 dose led to increased bee mortality in cage tests. Bees were also reluctant to take Met100-enriched sugar syrup, while the amount of uptake of Met25-enriched syrup was comparable to the control, where pure sugar syrup was administered.

### Example VIII - Positive effects of metformin on bee condition and overwintering - in vivo tests - on bees - field tests

### Apiary tests 1

Apiary tests were conducted as described in Example VI.

### The level of nosema in bee colonies

In the spring, a standard inspection of bee colonies was also carried out and the degree of nosema infestation of bee colonies was checked (Figs. 35 - 36). A decrease in infestation was observed in the groups in which the product was administered compared to the control - bee colonies in which the product was not administered.

### Apiary tests 2

The experiment used 10 hives, divided into two groups (control and test - 5 hives each). Families, of similar strength, occupying the majority of the nest were selected for the experiments. The bee queens (breed *- Car)* in the colonies were from the same year, and the hives were set up on the same apiary - a stationary apiary. The bee colonies used for the experiment are planted in a single-walled wooden hive with a hygienic bottom, Wielkopolska type.

To the bee colonies, at the end of August the feeding procedure was started in the form of invert feed, in which the test substance was administered at a ratio of 252 mg for every 21 of sugar syrup (concentration of 126 mg/dm³⁾ . Feeding was performed in 6 repetitions. The feed was topped up, depending on the rate of feed intake by the bee colony.

During the autumn administration of the substance, along with the feed, observations were made recording the number of frames sown and the number of larvae. The observations were made on two dates, the first before the substance was administered, and the second during the final fall inspection. While the number of larvae in both groups was decreasing, which is natural at this time of year, in the case of the test group with the substance, the colonies finished larvaeing faster. This was observed during the last rapid fall viewing on November 18. In the control colonies there was one frame of larvae each with an area of about 1 - 2 dm². There were no larvae in the test families.

The bee colonies were overwintered on two nests because the number of bees manning the frames in the hive required it. The decision to overwinter the colonies on a double nest was due to the increased number of bees and concerns about the occurrence of starvation in these colonies. The control colonies were overwintered like most colonies in the apiary on a single nest with an average number of frames overwintered of 6 units. In contrast, all test families were overwintered on a double nest with an average number of frames of 10 pc.

From the results, it can be concluded that colonies in the control and test groups did not differ significantly in the average number of frames with larvae during the additional inspection during feeding. However, something caused the families in the test group to have an increased number of bees, during the last inspection. One can hypothesize that these colonies did not lose bees during autumn development. By lost can be understood, for example, increased mortality caused by a number of factors.

The strength of bee colonies during the spring evaluation was assessed at two dates: 29.02 and 10.04. These two dates were strongly influenced by the weather window in the spring as well as more important inspections to prepare the bees for the season. During the first inspection, the colonies in the study group manned a higher number of frames, were more mobile, as well as had more larvae in the nest. The average number of slices with larvae at that time was 3 slices per hive. In the hives it was also possible to notice quite a substantial stock of winter food. Families in the control group stocked fewer combs and also had more modest winter food supplies. The average number of larvae patches was also lower, at 2.

The increased number of larvae patches in the study group was most likely related to the higher number of bees in the hive - the frames being manned. A larger number of bees can heat more larvae, as well as have more resources tied up in spring pollen collection, as well as bringing water to the hive. Families in the control group were smaller, but fledged just as well during spring sunshine flashes. It was found that the development of colonies within the test group was due to the administration of the substance during the autumn feeding of bee colonies.

At the time of the second inspection, the bee colonies of the test group already had an average of 6 larvae patches. The problem in the further development of colonies in the test group was that there was too much winter stock, so that the two outermost combs with food were each subtracted, and supplemented with dried combs. The families were very densely stocking all the slices at this time. Families in the control group were occupying an average of 8 slices at the time, and during this review it was decided to top up the nest to its full size (10 slices). However, they were weaker than the colonies in the study group. They had an average of 5 larvae patches on 10.04.

In surveys after applying the product, beekeepers emphasized its extremely positive effects on bee colonies, including increased larvae, increased number of adults, better condition of bees after overwintering, increased productivity, reduced morbidity, stronger bee flights in the spring, and higher bee tolerance to crop protection products such as insecticides. Noteworthy is the fact that bee colonies that flew such crops as rapeseed showed less sensitivity to the insecticides used on these crops, i.e. acetamiprid-based preparations: Carnadine, Inazuma, Kestrel, Mospilan and Avaunt, or pyrethroids. e.g. Decis, Bulldock, Mavrick Vita, or preparations based on a combination of pyrethroid and acetamiprid, i.e. D-Act, Inazuma, Carnadine + Kaiso or Mospilan with Sumicidin. The schedule of protective treatments starts from the main shoot growth stage, focusing initially on stem weevil control. In cooler weather, pyrethroids (e.g., Decis, Mavrick Vita) or their combinations with acetamiprid are effective. For strawberry control, Avaunt is recommended, while Mavrik Vita or preparations with acetamiprid work well for pimpleweed and stem weevil. These preparations are also highly toxic to the honey bee (Capela et al. 2022, Liu et al. 2022). After field tests, the following observations were made:
1. The colonies in the study group did not see a drastic decline in the winter bee population during the spring replacement of generations of individuals in the hive.
2. In colonies in the control group, a sharp decline in the number of bees was noted winter in the hive.
3. The autumn administration of the substance to the hive resulted in stronger colonies overwintering - more bees were observed in the hive than in the control group.
4. In the spring, there were more bees in the test group, more larvae than In the control group.

### Example IX - The preparation, veterinary composition and feed additive

In the experimental apiary, the agent was administered to two bee colonies. The agent can be a preparation according to the invention, a veterinary composition according to the invention or a feed additive according to the invention. The agent was administered in the following groups:
1. Bee colony 1, hive No. 5, the bee family was given 157.5 mg of metformin dissolved in 500 ml of sugar syrup (1:1) (concentration: 315 mg/dm³⁾ ;
2. Bee colony 2, hive No. 77, the bee family was given 1,575 mg of Met-CD complex dissolved in 500 ml of sugar syrup (1:1) (concentration: 3.15 g/dm³⁾ .

### NGS analysis

Next-Generation Sequencing (NGS) technology makes it possible to determine the gut microbiome of honey bees. Initially, total DNA was isolated using Qiagen's DNeasy Blood & Tissue Kit, then samples were prepared for NGS reads.

Metagenomic analysis of bacterial and archaeal populations was carried out based on the hypervariable V3-V4 region of the 16S rRNA gene. Specific primer sequences 341F and 785R (16S analysis) were used to amplify the selected region and prepare the library. PCR was performed using Q5 Hot Start High-Fidelity 2X Master Mix, reaction conditions according to the manufacturer's recommendations. Sequencing was performed on a MiSeq, paired-end (PE), 2x300nt, using Illumina's v3 kit.

The analysis consisted of two steps: automatic demultiplexing of samples, generation of fastq files containing raw reads. Bioinformatics analysis ensuring classification of the reads to the species level was carried out with the QIIME 2 software package based on the Silva 138 reference sequence database. The DADA2 package was also used, which allowed to distinguish sequences of biological origin from those newly created during the sequencing process. This package was also used to extract unique sequences of biological origin, the so-called *amplicon sequence variant* (ASV) sequences. The analysis consisted of steps:
1. Quality control of readings:
   - Error profile analysis of individual samples and dynamic generation of parameters for quality control (FIGARO tool);
   - Conduct quality control based on the *maximum expected* error rate of the sample (MER).
2. Data preprocessing using the Cutadapt tool:
   - removal of adaptor sequences;
   - Rejection of readings that are too short (minimum length of 30 nt).
3. Selecting unique ASV sequences (using the DADA2 package) by:
   - Filtering out sequences containing errors created during the sequencing process *(denoising);*
   - Combining paired reads - in order to increase the accuracy of sequencing, it is carried out in paired-end mode, so that at later stages of analysis the corresponding forward and reverse reads must be combined;
   - dereplication - fusion of identical, unique sequences while maintaining their number of occurrences and quality profile;
   - Chimera filtering - getting rid of constructs created from incorrect sequence assembly during PCR.
4. Assigning taxonomy to ASV sequences based on the Silva reference database, using a hybrid approach:
   - Comparison of ASV sequences against a base in search of identical reference sequences (vsearch);
   - unusual sequences left over from the previous step are classified based on methods using machine learning (sklearn).
5. creation of a phylogenetic tree
   - Performing alignment using the MAFFT algorithm;
   - Phylogenetic tree construction using the FastTree method.

Additional extended bioinformatics analysis was performed with R and using the packages phyloseq and vegan. Graphs were generated using the packages, gglpot2, gplots, plotly, metacoder and heatmaply.

### Summary of results

The gut microbiome plays a key role in maintaining homeostasis in animals and humans. In honey bees, the microbiome influences their nutrition and immune function. The gut microflora of the honeybee is remarkably cohesive and in healthy individuals is dominated by five ubiquitous, highly coevolving phylotypes representing >95% of all bacterial symbionts; *Lactobacillus, Alphaproteobacteria, Betaproteobacteria,* Gammaproteobacteria and *Bifidobacterium.* Recently, the link between the microbiome and the physiology of worker bees, including the expression of insulin-like peptides and vitellogenin, has also been highlighted (Engel et al, 2016; Maes et al, 2016; Ptaszynska et al, 2021).

The honeybee's gut microbiome includes five to nine taxa, each corresponding to a species or group of closely related species. Usually two clusters *of Lactobacillaceae* species collectively referred to as *"Lactobacillus* Firm- 4" *(L. mellis* and *L. mellifera)* and *"Lactobacillus* Firm-5" *(L. apis, L. helsingborgensis, L. kullabergensis, L. kimbladii* and *L. melliventris),* followed by *Bifidobacterium* spp. *(B. asteroides, B. coryneforme and B. indicum);* "Gamma-1" (*Gilliamella apicola)* and "Gamma-2" *(Frischella perrara);* "Alpha-1" *(Bartonella apis),* "Alpha-2.1" (Acetobacteraceae) and alpha-2.2" *(Parasaccharibacter apium* or *Bombella apis)* (Alberoni et al., 2021). The group of control bees had a microbiome composed mainly of bacteria belonging to *Klebsiella oxytoca* and *Enterobacter hormaechei,* with lesser amounts of bacteria belonging to the genera *Lactobacillus* and *Snodgrasella.*

After administration of the agents, the composition of the microbiome changed. Firstly, the bacterial microfolora was more diverse, and secondly, bacteria that are characteristic of the microbiome of healthy bees, i.e. various species of bacteria from the genus *Lactobacillus* and also *Snodgrasella alvi* and *Frischella perrera,* predominated.

*Snodgrassella alvi* affects the expression of immune genes in honey bees. Both live and heat-killed *S. alvi* protect honey bees from their opportunistic pathogen *Serratia marcescens* and lead to the expression of antimicrobial peptides in the honey bee (Horak et al., 2020; Martinson et al., 2012).

*Frischella perrara* causes strong activation of the host immune system. The bacterium activates antimicrobial peptide pattern recognition receptors and transporter genes, a melanization cascade. *F. perrara* also stimulates the immune system under hive conditions in the presence of other gut bacterial species (Emery et al., 2017, Engel et al., 2015).

The integrity of honey bees' intestinal microflora is important for the health and functionality of these pollinators. An increase in the diversity and abundance of bacteria of the genus *Lactobacillus,* such as *Lactobacillus apis, Lactobacillus helsingborgensis, Lactobacillus melliventris,* was observed after administration. These bacteria are combined with the intestinal microbiome of healthy bees which indicates that the product has a positive and protective effect on the honey bee. *Lactobacillus* bacteria have a beneficial effect on bee health and reduce the occurrence of pathogens. The addition of the *Lactobacillus* mixture to bee larvae food leads to a reduction of infections in the bee colony (Royan 2019). In addition, the administration of the product increased the share of batteries such as *Snodgrasella alvi* and *Frischella perrera* in the microbiome, which has a protective effect and supports the activation of the bees' immune system. It was also noted that there was an improvement in the condition of the bees in the test group, which was exposed to constant contact with agents plant protection (e.g., herbicides, insecticides). Based on the analysis of the research, the following beneficial effects of the feeding preparation, veterinary composition and feed additive according to the invention can be distinguished:
Condition and development of families:
   - Increases colony strength after overwintering by stimulating the bees' immune system,
   - Supports larvae development and increases larvae survival,
   - Improves the condition of bee queens, resulting in better larvae. Immunity and defence functions:
   - Raises the activity of immune genes,
   - Strengthens non-specific humoral immunity,
   - Increases the synthesis of antimicrobial peptides (e.g., apidins). The microbiome and digestion:
   - Supports the development of beneficial intestinal microflora,
   - Improves nutrient utilization,
   - Increases the biodiversity of symbiotic bacteria.
Detoxification and protection:
   - Supports detoxification processes after exposure to pesticides,
   - Reduces the negative effects of herbicides on the body of bees,
   - Reduces oxidative stress caused by xenobiotics.
Productivity:
   - Increases the honey yield of families,
   - Improves pollen and nectar harvesting efficiency,
   - Strengthens the flight activity of bee gatherers. Health prevention:
      - Reduces susceptibility to bacterial and fungal diseases,
      - Reduces bee mortality during the overwintering period,
      - Strengthens the natural protective barrier of the digestive tract. Based on the study, the following conclusions were made:
         1. The preparation increased the productivity of bee colonies. Colonies showed an increase in honey productivity of up to 15% in colonies given preparation A and 10% for colonies given preparation AB.
         2. The product has a protective effect, protecting bees from pathogens such as *Nosema* spp., Trypanosomatida and *Neogregarinorida, Ascosphaera apis and Paenibacillus* larvae. Studies show an unequivocal positive effect for Nosema spp., for other pathogens the protective effect of the product on bee colonies is time-limited and observable, within a short period of time after administration, i.e. up to 1 - 2 months after the application).
         3. An unequivocal positive effect on the honeybee microbiome. After administration of the preparations, the bacterial microfolora was more diverse and was dominated by bacteria that are characteristic of the microbiome of healthy bees, i.e. bacterial species of the genus *Lactobacillus,* in particular species: *L. apis, L. helsingborgensis* and *L. melliventris,* as well as *Snodgrasella alvi and Frischella perrera.* These bacteria are characteristic of healthy bees, which confirms the positive effect of the product on the condition of the insects.
         4. Observations by beekeepers confirm that the product protected the honey bee from the toxic effects of these insecticides.
         5. By extrapolating the results, it can be assumed that the preparation will have an equally positive effect on wild-island pollinating insects. Despite the enormous diversity of life forms and adaptive strategies within the *Hymenoptera,* from solitary wild bees to social ants to parasitic caterpillars, the basic physiological processes remain remarkably similar in them. This evolutionary legacy means that preparations to boost immunity or improve the condition of the honey bee can be just as effective in other representatives of the hymenoptera.

**The agent was also administered to *Bombyx mori L., Antheraea pernyi,* and *Bombus lucorum* also obtaining an identical agent effect to that of the honeybee.**

### Example X - The preparation, veterinary composition, and feed additive for animals of the family Apidae, Bombycidae or Saturniidae

The preparation, veterinary composition and feed additive may contain metformin, its salt (e.g., metformin hydrochloride (MetCl)) or its complex (e.g., metformin and cyclodextrin complex, metformin and heptakis(2,3,6-tris-O-methyl)cyclodextrin complex). The preparation, veterinary composition and additive according to the invention can be administered either in a single dose or repeatedly. **Tables 2** - **4** show the composition of the preparation, veterinary composition and additive according to the invention.

**Table 2. Composition of the preparation, veterinary composition and additive according to the invention**

| **Metformin or its complex or/and salt** | **Met** | | | | | | **Met-Cl** | | | | | |
|---|---|---|---|---|---|---|---|---|---|---|---|---|
| **Attractant** | **Concentration expressed in mg metformin/dm³** | | | | | | | | | | | |
| invert syrup | 12,6 | 664,6 | 129,2 | 323 | 6646 | 66460 | 12,6 | 664,6 | 129,2 | 323 | 6646 | 66460 |
| royal jelly | 12,6 | 664,6 | 129,2 | 323 | 6646 | 66460 | 12,6 | 664,6 | 129,2 | 323 | 6646 | 66460 |
| sucrose | 12,6 | 664,6 | 129,2 | 323 | 6646 | 66460 | 12,6 | 664,6 | 129,2 | 323 | 6646 | 66460 |
| fructose | 12,6 | 664,6 | 129,2 | 323 | 6646 | 66460 | 12,6 | 664,6 | 129,2 | 323 | 6646 | 66460 |
| glucose | 12,6 | 664,6 | 129,2 | 323 | 6646 | 66460 | 12,6 | 664,6 | 129,2 | 323 | 6646 | 66460 |
| sugar syrup | 12,6 | 664,6 | 129,2 | 323 | 6646 | 66460 | 12,6 | 664,6 | 129,2 | 323 | 6646 | 66460 |
| invert syrup | 12,6 | 664,6 | 129,2 | 323 | 6646 | 66460 | 12,6 | 664,6 | 129,2 | 323 | 6646 | 66460 |

| **Metformin or its complex or/and salt** | **Met-ME** | | | | | | **Met-CD** | | | | | |
|---|---|---|---|---|---|---|---|---|---|---|---|---|
| **Attractant** | **Concentration expressed in mg metformin/dm³** | | | | | | | | | | | |
| sugar paste | 12,6 | 664,6 | 129,2 | 323 | 6646 | 66460 | 12,6 | 664,6 | 129,2 | 323 | 6646 | 66460 |
| honey- sugar cake (kand). | 12,6 | 664,6 | 129,2 | 323 | 6646 | 66460 | 12,6 | 664,6 | 129,2 | 323 | 6646 | 66460 |
| bee pollen | 12,6 | 664,6 | 129,2 | 323 | 6646 | 66460 | 12,6 | 664,6 | 129,2 | 323 | 6646 | 66460 |
| honey | 12,6 | 664,6 | 129,2 | 323 | 6646 | 66460 | 12,6 | 664,6 | 129,2 | 323 | 6646 | 66460 |
| feather | 12,6 | 664,6 | 129,2 | 323 | 6646 | 66460 | 12,6 | 664,6 | 129,2 | 323 | 6646 | 66460 |

**Table 3. Composition of the preparation, veterinary composition and additive according to the invention**

| **Metformin or its complex or/and salt** | **Met-ME** | | | | | | **Met-CD** | | | | | |
|---|---|---|---|---|---|---|---|---|---|---|---|---|
| **Attractant** | **Concentration expressed in mg metformin/dm³** | | | | | | | | | | | |
| invert syrup | 12,6 | 664,6 | 129,2 | 323 | 6646 | 66460 | 12,6 | 664,6 | 129,2 | 323 | 6646 | 66460 |
| royal jelly | 12,6 | 664,6 | 129,2 | 323 | 6646 | 66460 | 12,6 | 664,6 | 129,2 | 323 | 6646 | 66460 |
| sucrose | 12,6 | 664,6 | 129,2 | 323 | 6646 | 66460 | 12,6 | 664,6 | 129,2 | 323 | 6646 | 66460 |
| fructose | 12,6 | 664,6 | 129,2 | 323 | 6646 | 66460 | 12,6 | 664,6 | 129,2 | 323 | 6646 | 66460 |
| glucose | 12,6 | 664,6 | 129,2 | 323 | 6646 | 66460 | 12,6 | 664,6 | 129,2 | 323 | 6646 | 66460 |
| sugar syrup | 12,6 | 664,6 | 129,2 | 323 | 6646 | 66460 | 12,6 | 664,6 | 129,2 | 323 | 6646 | 66460 |
| invert syrup | 12,6 | 664,6 | 129,2 | 323 | 6646 | 66460 | 12,6 | 664,6 | 129,2 | 323 | 6646 | 66460 |

| **Metformin or its complex or/and salt** | **Met** | | | | | | **Met-Cl** | | | | | |
|---|---|---|---|---|---|---|---|---|---|---|---|---|
| **Attractant** | **Concentration expressed in mg metformin/kg** | | | | | | | | | | | |
| sugar paste | 12,6 | 664,6 | 129,2 | 323 | 6646 | 66460 | 12,6 | 664,6 | 129,2 | 323 | 6646 | 66460 |
| honey- sugar cake (kand). | 12,6 | 664,6 | 129,2 | 323 | 6646 | 66460 | 12,6 | 664,6 | 129,2 | 323 | 6646 | 66460 |
| bee pollen | 12,6 | 664,6 | 129,2 | 323 | 6646 | 66460 | 12,6 | 664,6 | 129,2 | 323 | 6646 | 66460 |
| honey | 12,6 | 664,6 | 129,2 | 323 | 6646 | 66460 | 12,6 | 664,6 | 129,2 | 323 | 6646 | 66460 |
| feather | 12,6 | 664,6 | 129,2 | 323 | 6646 | 66460 | 12,6 | 664,6 | 129,2 | 323 | 6646 | 66460 |

**Table 4. Composition of the preparation, veterinary composition and additive according to the invention**

| **Metformin or its complex or/and salt** | **Met-ME and Met-CD (1:3) (w/w)** | | | | | | **Met-CD and Met (1:2) (w/w)** | | | | | |
|---|---|---|---|---|---|---|---|---|---|---|---|---|
| **Attractant** | **Concentration expressed in mg metformin/dm³** | | | | | | | | | | | |
| invert syrup | 12,6 | 664,6 | 129,2 | 323 | 6646 | 66460 | 12,6 | 664,6 | 129,2 | 323 | 6646 | 66460 |
| royal jelly | 12,6 | 664,6 | 129,2 | 323 | 6646 | 66460 | 12,6 | 664,6 | 129,2 | 323 | 6646 | 66460 |
| sucrose | 12,6 | 664,6 | 129,2 | 323 | 6646 | 66460 | 12,6 | 664,6 | 129,2 | 323 | 6646 | 66460 |
| fructose | 12,6 | 664,6 | 129,2 | 323 | 6646 | 66460 | 12,6 | 664,6 | 129,2 | 323 | 6646 | 66460 |
| glucose | 12,6 | 664,6 | 129,2 | 323 | 6646 | 66460 | 12,6 | 664,6 | 129,2 | 323 | 6646 | 66460 |
| sugar syrup | 12,6 | 664,6 | 129,2 | 323 | 6646 | 66460 | 12,6 | 664,6 | 129,2 | 323 | 6646 | 66460 |
| invert syrup | 12,6 | 664,6 | 129,2 | 323 | 6646 | 66460 | 12,6 | 664,6 | 129,2 | 323 | 6646 | 66460 |

| **Metformin or its complex or/and salt** | **Met and Met-ME (1:1) (w/w)** | | | | | | **Met-Cl and Met (1:4) (w/w)** | | | | | |
|---|---|---|---|---|---|---|---|---|---|---|---|---|
| **Attractant** | **Concentration expressed in mg metformin/kg** | | | | | | | | | | | |
| sugar paste | 12,6 | 664,6 | 129,2 | 323 | 6646 | 66460 | 12,6 | 664,6 | 129,2 | 323 | 6646 | 66460 |
| honey- sugar cake (kand). | 12,6 | 664,6 | 129,2 | 323 | 6646 | 66460 | 12,6 | 664,6 | 129,2 | 323 | 6646 | 66460 |
| bee pollen | 12,6 | 664,6 | 129,2 | 323 | 6646 | 66460 | 12,6 | 664,6 | 129,2 | 323 | 6646 | 66460 |
| honey | 12,6 | 664,6 | 129,2 | 323 | 6646 | 66460 | 12,6 | 664,6 | 129,2 | 323 | 6646 | 66460 |
| feather | 12,6 | 664,6 | 129,2 | 323 | 6646 | 66460 | 12,6 | 664,6 | 129,2 | 323 | 6646 | 66460 |

### Example XI - Evaluation of metformin residues in bee honey samples

### Sample preparation for analysis

The research used a test procedure based on PN-EN 15662:2018-06 "Food of plant origin - Multimethod for the determination of pesticide residues using GC and LC-based analysis after extraction/division with acetonitrile and purification by dispersive SPE - QuEChERS modular method." Into a centrifuge tube of 50 ml was weighed ±1 g of honey sample, 5 ml of water was added and shaken for 15 min on a platform shaker. Then 10 ml of acetonitrile was added and shaken again for 10 minutes. Buffer mixture (4 g anhydrous magnesium sulfate+ 1 g sodium chloride + 1 g trisodium citrate dihydrate + 0.5 g disodium hydrogen citrate one and a half (Citrate Extraction Tube), Supelco) was added and shaken for 3 minutes on a Multi Reax shaker (Heidolph). The extract was centrifuged for 3.5 minutes at 4,500 rpm at 15°C. 0.1 ml of supernatant was taken from the solution and transferred to a test tube, 0.9 ml of LC phase (PHASE A and PHASE B, 95:5 v:v) was added. The solution was filtered through a syringe filter directly into an autosampler vial. The resulting solution was a 100-fold dilution of the starting extract (1 ml of solution contained 0.01 g of sample).

### Conditions for instrumental analysis

LC-MS/MS kit - Eksigent expert ultraLC 100-XL chromatograph (Eksigent Technologies, Dublin, USA) with QTRAP 6500 mass spectrometer (AB Sciex Instruments, Foster City, USA) controlled by Analyst Software version 1.6.2 (AB Sciex). Data collection in multiple reaction monitoring (MRM) mode. MultiQuant Software version 3.0.2 (AB Sciex) was used for quantitative evaluation.

### Results

Samples taken in autumn contained metformin from below the limit of quantification to 0.346 µg/g. In contrast, samples taken in the spring contained metformin from values below the limit of quantification to 0.281 µg/g. In contrast, the metformin content of the control group ranged from below the limit of quantification to 0.008 µg/g during both study periods.

### Applications

In honey samples, the metformin content found is acceptable. After the autumn administration of metformin, in the spring harvest its labelled residues are present at a lower level (about 23%) compared to the content of metformin in honey in the autumn harvest.

## Claims

1. A preparation for the feeding of animals of the family *Apidae, Bombycidae* or *Saturniidae* containing metformin or/and its physiologically acceptable complex or/and salt.

2. The preparation according to claim. 1, **characterised in that it** contains a complex of metformin and cyclodextrin or/and a complex of metformin and heptakis(2,3,6-tris-O- methyl)cyclodextrin or/and metformin hydrochloride.

3. The preparation according to claim. 1 or 2, **characterised in that** the animals belong to the genera *Apis, Bombus, Psithyrus, Bombyx, Antheraea,* favorably animals of the species *Apis melifera, Apis cerana, Apis dorsata, Apis florea, Bombyx mori L., Antheraea pernyi.*

4. The preparation according to any of the claims 1 - 3, **characterised in that** it contains at least one attractant selected from the group consisting of: bee pollen, royal jelly, honey, bee bread, sucrose solution, fructose solution, glucose solution, sugar syrup, invert syrup, sugar paste, honey-sugar cake (cand.).

5. The preparation according to any of claims 1 - 4, **characterised in that** the concentration of metformin in its physiologically acceptable complex or salt is up to 6.5 g metformin/dm³ of liquid attractant or up to 6.5 g metformin/kg of solid attractant.

6. A veterinary composition containing an active substance and veterinary acceptable carriers, **characterised in that** the active substance is metformin or a veterinary acceptable complex or/and salt thereof for use in the treatment or prevention of nosema in animals of the families *Apidae, Bombycidae* or *Saturniidae* caused by the sporozoite *Vairimorpha (Nosema) apis. Vairimorpha (Nosema) ceranae, Nosema bombycis, Nosema bombi* or *Nosema pernyi.*

7. The composition according to claim. 6, **characterised in that** the active substance is a complex of metformin and cyclodextrin or/and a complex of metformin and heptakis(2,3,6-tris-O- methyl)cyclodextrin or metformin hydrochloride.

8. The composition according to claim. 6 or 7, **characterised in that** the animals belong to the genera *Apis, Bombus, Psithyrus, Bombyx, Antheraea,* advantageously animals of the species *Apis melifera, Apis cerana, Apis dorsata, Apis florea, Bombyx mori L., Antheraea pernyi.*

9. The composition according to any of the claims. 6 - 8, **characterised in that** the concentration of the active substance is up to 6.5 g metformin/dm³ of liquid attractant or up to 6.5 g metformin/kg of solid attractant.

10. The composition according to any of the claims. 6 - 9, **characterised in that** the acceptable carrier is at least one ingredient selected from the group consisting of: bee pollen, royal jelly, honey, bee bread, sucrose solution, fructose solution, glucose solution, sugar syrup, invert syrup, sugar paste, honey-sugar cake (cand.).

11. A feed additive for *Apidae, Bombycidae* or *Saturniidae* containing metformin or its physiologically acceptable complex or/and salt.

12. The additive according to claim. 11, **characterised in that it** contains a complex of metformin and cyclodextrin or/and a complex of metformin and heptakis(2,3,6-tris-O- methyl)cyclodextrin or metformin hydrochloride.

13. The additive according to claim. 11 or 12, **characterised in that** the animals belong to the genera *Apis, Bombus, Psithyrus, Bombyx, Antheraea,* preferably animals of the species *Apis melifera, Apis cerana, Apis dorsata, Apis florea, Bombyx mori L., Antheraea pernyi.*

14. The additive according to any of the claims 11 - 13, **characterised in that** it contains at least one attractant selected from the group consisting of: bee pollen, royal jelly, honey, bee bread, sucrose solution, fructose solution, glucose solution, sugar syrup, invert syrup, sugar paste, honey-sugar cake (cand.).

15. The additive according to any of claims 11 - 14, **characterised in that** the concentration of metformin in its physiologically acceptable complex or salt is up to 6.5 g metformin/dm³ of liquid attractant or up to 6.5 g metformin/kg of solid attractant.

16. A non-therapeutic method of promoting the abundance, post-wintering condition, number of broods, increasing productivity, increasing diversity and abundance of gut microbiome bacteria, activity of the immune system, promoting condition after contact with plant protection products, decreasing the incidence of infections in animals of the family *Apidae, Bombycidae* or *Saturniidae* by feeding them with a preparation according to any of the claims. 1 - 5, a veterinary composition according to claims. 6 - 10 or a feed additive according to claims 11 - 15.

17. The non-therapeutic method according to claim. 16, **characterised in that** the preparation, composition or additive is administered once or repeatedly.

18. The non-therapeutic method according to claim. 16 or 17, **characterised in that** it increases the honey yield of animals of the family *Apidae.*
